# EUROPÄISCHE PATENTSCHRIFT

(11) **EP 0 669 328 B1**
(45) Veröffentlichungstag und Bekanntmachung des Hinweises auf die Patenterteilung: **30.09.1998**
(21) Anmeldenummer: 95810018.2
(22) Anmeldetag: 10.01.1995
(51) Int. Cl.: C07D 295/03, C09D 5/08

(54) **Komplexe von Morpholinderivaten mit Ketocarbonsäuren als Korrosionsinhibitoren**
Complexes of morpholine derivatives and ketocarboxylic acids as corrosion inhibitors
Complexes de dérivés de morpholine et d'acides cétocarboxyliques comme inhibiteurs de corrosion

(30) Priorität: 18.01.1994 CH 149/94
(43) Veröffentlichungstag der Anmeldung: 30.08.1995
(73) Patentinhaber: Ciba Specialty Chemicals Holding Inc., 4057 Basel (CH)
(72) Erfinder: Kramer, Dr. Andreas, CH-3186 Düdingen (CH); Braig, Dr. Adalbert, D-79589 Binzen (DE)

(56) Entgegenhaltungen:
- EP-A- 0 041 927
- EP-A- 0 144 663
- EP-A- 0 412 933
- EP-A- 0 496 555
- INDIAN J. TECHNOL., Bd. 8, Nr. 3, 1970 Seiten 98-100, R. NATARAJAN, A. D. PUROHIT 'Substituted Benzoates as Corrosion Inhibitors for Ferrous & Non-ferrous Metals in Neutral Solutions'

## Beschreibung

Die vorliegende Erfindung betrifft Komplexe von Morpholinderivaten mit Ketocarbonsäuren, Ueberzugszusammensetzungen enthaltend ein organisches filmbildendes Bindemittel, bevorzugt ein Anstrichmittel und die neuen Korrosionsinhibitoren, sowie die Verwendung derselben in Ueberzugszusammensetzungen zum Schutz von metallischen Oberflächen.

Die Verwendung von Alkali- Ammonium- und Amin-Salzen von Ketocarbonsäuren als Korrosionsinhibitoren in wässrigen Systemen ist bekannt und beispielsweise in US-A-4 909 987, US-A-5 128 396 oder EP-A-496 555 beschrieben.

Aus EP-A-300 325, Beispiel 7, ist zu entnehmen, dass die Umsetzung von einem Aequivalent 3-Benzoylpropionsäure mit einem Aequivalent Morpholin in Ethanol ein kristallines Salz liefert, dessen Zusammensetzung erwartungsgemäss aus einem Teil Säure und einem Teil Base besteht.

Es wurde nun gefunden, dass bei der Umsetzung einer Ketocarbonäure mit einem Morpholinderivat ohne Lösungsmittel oder in Gegenwart eines aprotischen Lösungsmittels kristalline Komplexe erhalten werden, deren Zusammensetzung aus zwei Teilen Säure und nur einem Teil Base besteht. Diese kristallinen Komplexverbindungen eignen sich besonders gut als Korrosionsinhibitoren in Ueberzugszusammensetzungen zum Schutz von metallischen Oberflächen aber auch zur Vorbehandlung metallischer Substrate. Die kristallinen Komplexverbindungen führen zu keiner Interaktion mit dem Lacksystem, insbesondere wässrigen Lacksystem, und zeichnen sich in Ueberzugszusammensetzungen durch eine ausgezeichnete Lagerstabilität aus. Sie sind ebenso geeignet für den temporären wie auch für den permanenten Rostschutz. Diese Lacksysteme zeichnen sich zudem durch eine ausgezeichnete Nasshaftfestigkeit aus.

Die vorliegende Erfindung betrifft daher kristalline Komplexverbindungen der Formel I 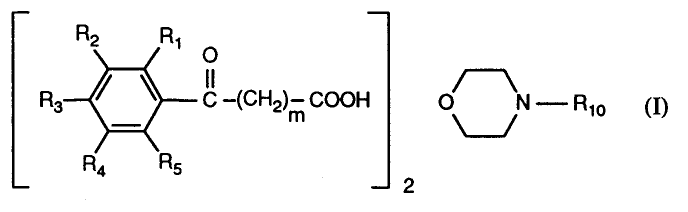 worin R₁, R₂, R₃, R₄ und R₅ unabhängig voneinander Wasserstoff, Halogen, Nitro, Cyano, CF₃, C₁-C₁₅-Alkyl, C₅-C₁₂-Cycloalkyl, C₂-C₁₅-Alkenyl, C₁-C₁₂-Halogenalkyl, C₁-C₁₂-Alkoxy, C₁-C₁₂-Alkylthio, unsubstituiertes oder durch C₁-C₄-Alkyl substituiertes C₆-C₁₀-Aryl; unsubstituiertes oder durch C₁-C₄-Alkyl substituiertes C₆-C₁₀-Aryloxy; unsubstituiertes oder am Arylrest durch 1 bis 3 C₁-C₄-Alkyl substituiertes C₇-C₁₂-Arylalkyl; -CO₂R₆, -COR₆ oder 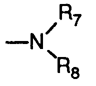 darstellen, wobei mindestens einer der Reste R₁ bis R₅ Wasserstoff, Halogen oder C₁-C₁₅-Alkyl bedeutet; ferner die Reste R₁ und R₂, R₂ und R₃, R₃ und R₄ oder R₄ und R₅ zusammen mit den Kohlenstoffatomen, an die sie gebunden sind, einen Benzo- oder Cyclohexenyl-Ring bilden,
R₆ C₁-C₂₀-Alkyl, durch Sauerstoff, Schwefel oder 〉N―R₉ unterbrochenes C₂-C₂₀-Alkyl;
unsubstituiertes oder am Arylrest durch 1 bis 3 C₁-C₄-Alkyl substituiertes C₇-C₁₂-Arylalkyl bedeutet,
R₇ und R₈ unabhängig voneinander Wasserstoff, C₁-C₂₄-Alkyl oder durch Sauerstoff, Schwefel oder 〉N―R₉ unterbrochenes C₂-C₂₄-Alkyl darstellen,
R₉ Wasserstoff oder C₁-C₈-Alkyl bedeutet,
R₁₀ C₁-C₁₅-Alkyl, durch Sauerstoff, Schwefel oder 〉N―R₉ unterbrochenes C₂-C₂₀-Alkyl; C₅-C₁₂-Cycloalkyl, C₂-C₁₅-Alkenyl, C₁-C₁₂-Alkoxy, C₁-C₁₂-Alkylthio, unsubstituiertes oder durch C₁-C₄-Alkyl substituiertes C₆-C₁₀-Aryl; unsubstituiertes oder durch C₁-C₄-Alkyl substituiertes C₆-C₁₀-Aryloxy; unsubstituiertes oder am Arylrest durch 1 bis 3 C₁-C₄-Alkyl substituiertes C₇-C₁₂-Arylalkyl darstellt, und
m eine ganze Zahl aus dem Bereich von 2 bis 5 bedeutet.

Halogen bedeutet beispielsweise Fluor, Chlor, Brom oder Iod. Bevorzugt sind Fluor, Chlor oder Brom, insbesondere Chlor oder Brom.

Alkyl mit bis zu 24 Kohlenstoffatomen bedeutet einen verzweigten oder unverzweigten Rest wie beispielsweise Methyl, Ethyl, Propyl, Isopropyl, n-Butyl, sec-Butyl, Isobutyl, tert-Butyl, 2-Ethylbutyl, n-Pentyl, Isopentyl, 1-Methylpentyl, 1,3-Dimethylbutyl, n-Hexyl, 1-Methylhexyl, n-Heptyl, Isoheptyl, 1,1,3,3-Tetramethylbutyl, 1-Methylheptyl, 3-Methylheptyl, n-Octyl, 2-Ethylhexyl, 1,1,3-Trimethylhexyl, 1,1,3,3-Tetramethylpentyl, Nonyl, Decyl, Undecyl, 1-Methylundecyl, Dodecyl, 1,1,3,3,5,5-Hexamethylhexyl, Tridecyl, Tetradecyl, Pentadecyl, Hexadecyl, Heptadecyl, Octadecyl, Eicosyl oder Docosyl. Bevorzugt ist Alkyl mit 1 bis 12, insbesondere 1 bis 8 Kohlenstoffatomen. Eine bevorzugte Bedeutung von R₃ ist C₁-C₄-Alkyl, insbesondere Methyl. Eine bevorzugte Bedeutung von R₁₀ ist C₁-C₄-Alkyl, insbesondere Ethyl.

C₅-C₁₂-Cycloalkyl bedeutet beispielsweise Cyclopentyl, Cyclohexyl, Cycloheptyl, Cyclooctyl, Cyclodecyl oder Cyclodocecyl. Bevorzugt ist Cyclohexyl.

Alkenyl mit 2 bis 15 Kohlenstoffatomen bedeutet einen verzweigten oder unverzweigten Rest wie beispielsweise Vinyl, 2-Propenyl (Allyl), 2-Butenyl, 3-Butenyl, Isobutenyl, n-2,4-Pentadienyl, 3-Methyl-2-butenyl, n-2-Octenyl, n-2-Dodecenyl oder iso-Dodecenyl. Bevorzugt ist Alkenyl mit 3 bis 12, insbesondere 3 bis 8, z.B. 3 bis 6, vor allem 3 bis 4 Kohlenstoffatomen.

Halogenalkyl mit bis zu 12 Kohlenstoffatomen bedeutet einen verzweigten oder unverzweigten Rest wie beispielsweise Chlormethyl, Bromethyl, Fluorpropyl, Chlorpentyl, Chlorhexyl, Chloroctyl, Chlordecyl oder Chlordodecyl. Bevorzugt ist Halogenalkyl mit 3 bis 8, insbesondere 3 bis 6, Kohlenstoffatomen.

Alkoxy mit bis zu 12 Kohlenstoffatomen bedeutet einen verzweigten oder unverzweigten Rest wie beispielsweise Methoxy, Ethoxy, Propoxy, Isopropoxy, n-Butoxy, Isobutoxy, Pentoxy, Isopentoxy, Hexoxy, Heptoxy, Octoxy oder Decyloxy. Bevorzugt ist Alkoxy mit 1 bis 8, insbesondere 1 bis 4, Kohlenstoffatomen.

Alkylthio mit bis zu 12 Kohlenstoffatomen bedeutet einen verzweigten oder unverzweigten Rest wie beispielsweise Methylthio, Ethylthio, Propylthio, Isopropylthio, n-Butylthio, Isobutylthio, Pentylthio, Isopentylthio, Hexylthio, Heptylthio, Octylthio, Decylthio oder Dodecylthio. Bevorzugt ist Alkylthio mit 1 bis 8, insbesondere 1 bis 4, Kohlenstoffatomen.

Unsubstituiertes oder durch C₁-C₄-Alkyl substituiertes C₆-C₁₀-Aryl, das vorzugsweise 1 bis 3, insbesondere 1 oder 2 Alkylgruppen trägt, bedeutet beispielsweise Phenyl, Naphthyl, o-, m- oder p-Methylphenyl, 2,3-Dimethylphenyl, 2,4-Dimethylphenyl, 2,5-Dimethylphenyl, 2,6-Dimethylphenyl, 3,4-Dimethylphenyl, 3,5-Dimethylphenyl, 2-Methyl-6-ethylphenyl, 4-tert-Butylphenyl, 2-Ethylphenyl, 2,6-Diethylphenyl, 2-Methylnaphthyl, 1-Methylnaphthyl, 4-Methylnaphthyl, 2-Ethylnaphthyl oder 2,6-Diethylnaphthyl.

Unsubstituiertes oder durch C₁-C₄-Alkyl substituiertes C₆-C₁₀-Aryloxy, das vorzugsweise 1 bis 3, insbesondere 1 oder 2 Alkylgruppen trägt, bedeutet beispielsweise Phenoxy, Naphthoxy, o-, m- oder p-Methylphenoxy, 2,3-Dimethylphenoxy, 2,4-Dimethylphenoxy, 2,5-Dimethylphenoxy, 2,6-Dimethylphenoxy, 3,4-Dimethylphenoxy, 3,5-Dimethylphenoxy, 2-Methyl-6-ethylphenoxy, 4-tert-Butylphenoxy, 2-Ethylphenoxy, 2,6-Diethylphenoxy, 2-Methylnaphthoxy, 1-Methylnaphthoxy, 4-Methylnaphthoxy, 2-Ethylnaphthoxy oder 2,6-Diethylnaphthoxy.

Unsubstituiertes oder am Arylrest durch 1 bis 3 C₁-C₄-Alkyl substituiertes C₇-C₁₂-Arylalkyl bedeutet beispielsweise Phenyl-C₁-C₆-alkyl oder Naphthyl-C₁-C₂-alkyl wie etwa Benzyl, 4-Methylbenzyl, 4-tert-Butylbenzyl, 2,4-Dimethylbenzyl, α-Methylbenzyl, α,α-Dimethylbenzyl, 2-Phenylethyl, 2-Naphthylmethyl, 1-Naphtylmethyl, 1-Naphthylethyl oder 2-Naphthylethyl. Bevorzugt ist Benzyl.

Durch Sauerstoff, Schwefel oder 〉N―R₉ unterbrochenes Alkyl mit 2 bis zu 24 Kohlenstoffatomen bedeutet beispielsweise CH₃-O-CH₂-, CH₃-S-CH₂-, CH₃-NH-CH₂-, CH₃-N(CH₃)-CH₂-, CH₃-O-CH₂CH₂-O-CH₂-, CH₃-(O-CH₂CH₂-)₂O-CH₂-, CH₃-(O-CH₂CH₂-)₃O-CH₂- oder CH₃-(O-CH₂CH₂-)₄O-CH₂-.

Bevorzugt sind kristalline Komplexverbindungen der Formel I, worin mindestens zwei der Reste R₁ bis R₅ Wasserstoff sind.

m ist in Formel I bevorzugt 2 bis 4, insbesondere 2.

Besonders bevorzugt sind kristalline Komplexverbindungen der Formel I, worin
R₁ Wasserstoff bedeutet,
R₂, R₃, R₄ und R₅ unabhängig voneinander Wasserstoff, Chlor, Brom, Nitro, Cyano, CF₃, C₁-C₈-Alkyl, C₅-C₇-Cycloalkyl, C₃-C₈-Alkenyl, C₁-C₈-Alkoxy, C₁-C₈-Alkylthio, Phenyl, Phenoxy, Benzyl, -CO₂R₆, -COR₆ oder 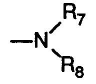 darstellen,
R₆ C₁-C₁₂-Alkyl, durch Sauerstoff unterbrochenes C₂-C₁₂-Alkyl; oder Benzyl bedeutet,
und
R₇ und R₈ unabhängig voneinander Wasserstoff, C₁-C₈-Alkyl oder durch Sauerstoff unterbrochenes C₂-C₁₂-Alkyl darstellen.

Auch besonders bevorzugt sind kristalline Komplexverbindungen der Formel I, worin
R₁₀ C₁-C₈-Alkyl, durch Sauerstoff oder Schwefel unterbrochenes C₃-C₁₂-Alkyl; C₅-C₇-Cycloalkyl, C₃-C₈-Alkenyl, C₁-C₈-Alkoxy, C₁-C₈-Alkylthio, Phenyl, Phenoxy oder Benzyl bedeutet.

Ebenfalls besonders bevorzugt sind kristalline Komplexverbindungen der Formel I, worin
R₁, R₂ und R₄ Wasserstoff bedeuten,
R₃ und R₅ unabhängig voneinander Wasserstoff, Chlor, Brom, CF₃, C₁-C₈-Alkyl, Cyclohexyl, C₁-C₈-Alkoxy, Phenyl, -CO₂R₆, -COR₆ oder 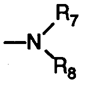 darstellen,
R₆ C₁-C₈-Alkyl bedeutet,
R₇ und R₈ unabhängig voneinander Wasserstoff oder C₁-C₈-Alkyl darstellen,
R₁₀ C₁-C₈-Alkyl, Cyclohexyl, Phenyl oder Benzyl darstellt, und
m eine ganze Zahl aus dem Bereich von 2 bis 4 bedeutet.

Von besonderem Interesse sind kristalline Komplexverbindungen der Formel I, worin
R₁, R₂, R₄ und R₅ Wasserstoff bedeuten, und m 2 ist.

Speziell von Interesse sind kristalline Komplexverbindungen der Formel I, worin R₁₀ C₁-C₄-Alkyl bedeutet.

Ebenfalls von Interesse sind kristalline Komplexverbindungen der Formel I, worin
R₁, R₂, R₄ und R₅ Wasserstoff sind,
R₃ Wasserstoff, C₁-C₄-Alkyl, Phenyl oder Chlor bedeutet,
R₁₀ C₁-C₄-Alkyl darstellt, und
m 2 bedeutet.

Ganz besonders bevorzugt ist der kristalline 2:1 Komplex der 3-(4-Methylbenzoyl)propionsäure mit N-Ethylmorpholin [Verbindung (101)].

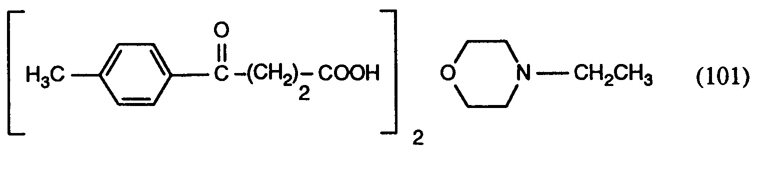

Von dieser Komplexverbindung (101) (Beispiel 1) wurde eine Röntgenstrukturanalyse durchgeführt. In Figur 1 ist die Kristallpackung und in Figur 2 die Numerierung der Atome in der Komplexverbindung (101) dargestellt. Die experimentellen Kristalldaten sind in Beispiel 1 zusammengefasst.

### Figur 1: Kristallpackung der Komplexverbindung (101)

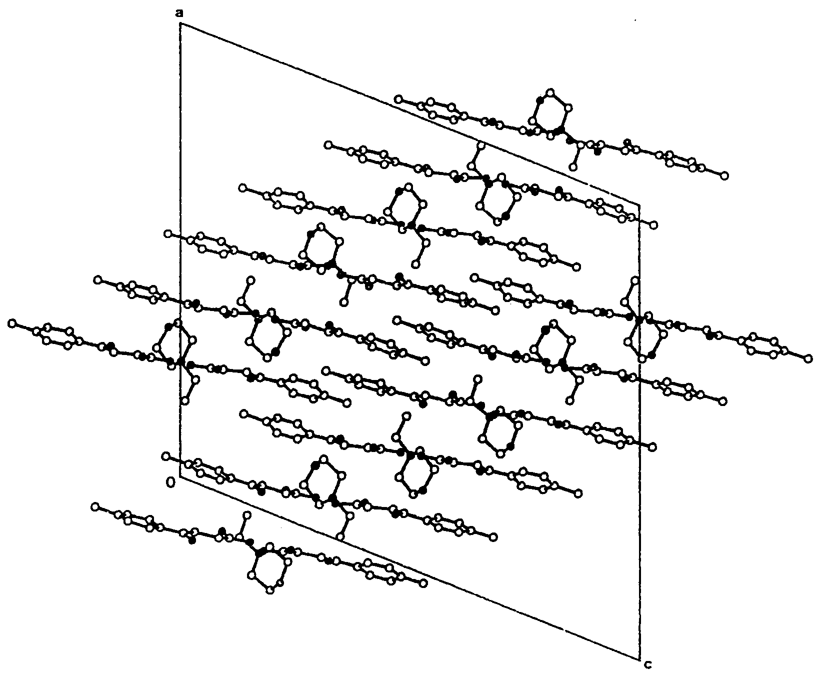

### Figur 2: Numerierung der Atome in der Komplexverbindung (101).

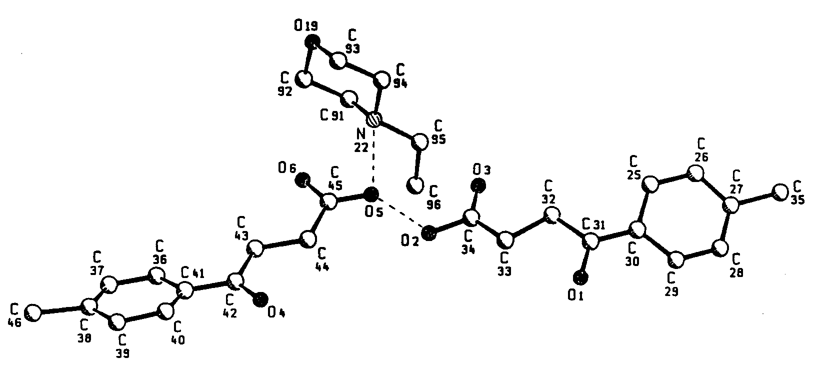

Die durchschnittlichen Bindungslängen in Ångstrom sind in Tabelle 1 zusammengefasst.

**Tabelle 1**

| Durchschnittliche Bindungslängen in Å | | | | | |
|---|---|---|---|---|---|
| O (1) | C (31) | 1,255 Å | O (2) | C (34) | 1,332 Å |
| O (3) | C (34) | 1,252 Å | O (4) | C (42) | 1,244 Å |
| O (5) | C (45) | 1,251 Å | O (6) | C (45) | 1,260 Å |
| O (19) | C (92) | 1,440 Å | O (19) | C (93) | 1,471 Å |
| N (22) | C (91) | 1,547 Å | N (22) | C (94) | 1,501 Å |
| N (22) | C (95) | 1,561 Å | C (25) | C (26) | 1,420 Å |
| C (25) | C (30) | 1,412 Å | C (26) | C (27) | 1,389 Å |
| C (27) | C (28) | 1,393 Å | C (27) | C (35) | 1,526 Å |
| C (28) | C (29) | 1,359 Å | C (29) | C (30) | 1,400 Å |
| C (30) | C (31) | 1,456 Å | C (31) | C (32) | 1,495 Å |
| C (32) | C (33) | 1,576 Å | C (33) | C (34) | 1,494 Å |
| C (36) | C (37) | 1,420 Å | C (36) | C (41) | 1,409 Å |
| C (37) | C (38) | 1,365 Å | C (38) | C (39) | 1,396 Å |
| C (38) | C (46) | 1,550 Å | C (39) | C (40) | 1,423 Å |
| C (40) | C (41) | 1,400 Å | C (41) | C (42) | 1,460 Å |
| C (42) | C (43) | 1,512 Å | C (43) | C (44) | 1,556 Å |
| C (44) | C (45) | 1,501 Å | C (91) | C (92) | 1,525 Å |
| C (93) | C (94) | 1,510 Å | C (95) | C (96) | 1,491 Å |

Zur Herstellung der erfindungsgemässen kristallinen Komplexverbindungen der Formel I ist in der Literatur kein Analogie-Verfahren beschrieben.

Ein weiterer Gegenstand der Erfindung ist daher auch ein neues Verfahren zur Herstellung der kristallinen Komplexverbindungen der Formel I, dadurch gekennzeichnet, dass eine Ketocarbonsäure der Formel II 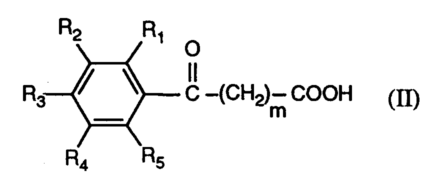 worin die allgemeinen Symbole wie in Formel I definiert sind, mit einem Morpholinderivat der Formel III 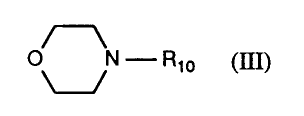 worin R₁₀ wie in Formel I definiert ist, ohne Lösungsmittel oder in Gegenwart eines aprotischen Lösungsmittels umgesetzt wird.

Für dieses Verfahren nicht geeignet sind protische Lösungsmittel wie Alkohole, beispielsweise Methanol oder Ethanol.

Bevorzugte Reaktionsbedingungen des erfindungsgemässen Verfahrens sind die folgenden.

Bevorzugt wird eine Mischung der Ketocarbonsäure der Formel II mit einem Morpholinderivat der Formel III bis zu einer homogenen und klaren Schmelze erwärmt. Nach dem Abkühlen auf Raumtemperatur kristallisieren die erfindungsgemässen Komplexverbindungen der Formel I aus. Diese werden anschliessend abfiltriert und mit einem geeigneten Lösungsmittel gewaschen. Nach dem Trocknen des Rückstandes werden die erfindungsgemässen kristallinen Komplexverbindungen der Formel I in analysenreiner Form erhalten.

Wie aus Beispiel 1, 2 und 3 ersichtlich, spielt es überraschenderweise bei diesem Verfahren keine Rolle in welchem molaren Verhältnis die Ketocarbonsäure der Formel II zum Morpholinderivat der Formel III eingesetzt wird. In jedem Fall wird der gleiche kristalline 2:1-Komplex der Formel I isoliert.

Bevorzugt wird jedoch ein Verfahren, dadurch gekennzeichnet, dass das molare Verhältnis der Ketocarbonsäure der Formel II zum Morpholinderivat der Formel m 4:1 bis 1:4, insbesondere 2:1 bis 1:2, z.B. 1:1, beträgt.

Die Umsetzung der beiden Komponenten erfolgt bevorzugt unter einer Inertgasatmosphäre, wie beispielsweise Stickstoff- oder Argon-Atmosphäre.

Die Reaktionstemperatur beziehungsweise die Schmelztemperatur im erfindungsgemässen Verfahren hängt von den physikalischen Eigenschaften der eingesetzten Ketocarbonsäure der Formel II und dem Morpholinderivat der Formel III ab. Bevorzugt wird eine Schmelztemperatur von 40 bis 130°C, insbesondere 50 bis 120°C. Eine speziell bevorzugte Schmelztemperatur zur Herstellung der besonders bevorzugten kristallinen Komplexverbindungen der Formel I beträgt 60 bis 80°C.

Sobald das Reaktionsgemisch die Schmelztemperatur erreicht und die Schmelze homogen und klar ist, wird bevorzugt vor dem Abkühlen nur noch 1 bis 20 Minuten, insbesondere 1 bis 10 Minuten, bei dieser Temperarur nachgerührt.

Die ausgefallenen kristallinen Komplexverbindungen der Formel I werden bei der Filtration bevorzugt mit einem leicht flüchtigen aprotischen Lösungsmittel aus der Reihe der Kohlenwasserstoffe, wie beispielsweise Ligroin, Pentan, Hexan, Hexan-Fraktionen oder Benzinen, gewaschen. Besonders bevorzugt ist Hexan.

Die erfindungsgemässen Verbindungen der Formel I sind bei Raumtemperatur stabil und können bei Normaldruck oder unter Hochvakuum getrocknet werden. Speziell bevorzugt werden die kristallinen Komplexverbindungen der Formel I in einem Trockenschrank mit Wasserstrahlvakuum bei einer Temperatur von 20 bis 30°C getrocknet.

Werden die erfindungsgemässen kristallinen Komplexverbindungen unter drastischeren Bedingungen, wie beispielsweise erhöhter Temperatur getrocknet, verflüchtigt sich das Morpholinderivat der Formel III teilweise und der erfindungsgemässe 2:1-Komplex wird teilweise zerstört. Das so getrocknete Produkt enthält nach wie vor teilweise die erfindungsgemässe kristalline Komplexverbindung der Formel I, ist aber stark mit der freien Ketocarbonsäure der Formel II verunreinigt.

Die Umsetzung der Ketocarbonsäure der Formel II mit einem Morpholinderivat der Formel III kann auch in Gegenwart eines aprotischen Lösungsmittels durchgeführt werden.

Geeignete aprotische Lösungsmittel sind die gleichen wie sie zum Waschen der Produkte Verwendung finden. Bevorzugte Lösungsmittel sind Kohlenwasserstoffe, wie beispielsweise Benzol, Toluol, Xylol, Ligroin, Pentan, Hexan, Hexan-Fraktionen oder Benzin. Besonders bevorzugt sind aliphatische Kohlenwasserstoffe, z.B. Hexan. Als aprotisches Lösungsmittel kann auch ein Ueberschuss der Verbindung der Formel III dienen.

Eine bevorzugte Reaktionstemperatur beim erfindungsgemässen Verfahren mit einem Lösungsmittel, wie beispielsweise einem Lösungsmittel aus der Reihe der Kohlenwasserstoffe, ist die Siedetemperatur des Lösungsmittels.

Die vorliegende Erfindung betrifft daher auch Produkte, erhältlich durch Umsetzung einer Ketocarbonsäure der Formel II mit einem Morpholinderivat der Formel III ohne Lösungsmittel oder in Gegenwart eines aprotischen Lösungsmittels.

Die Ketocarbonsäuren der Formel II sind bekannt und zum Teil im Handel erhältlich, oder können wie in Houben-Weyl, Methoden der Organischen Chemie, Band VIII, S. 381-382 (1952) und Band E5, S. 398-399 (1985) beschrieben, hergestellt werden. So liefert beispielsweise die Friedel-Crafts-Acylierung von substituierten Aromaten (Benzol- und Naphtalin-Derivate) mit cyclischen Anhydriden die Verbindungen der Formel II in ausgezeichneten Ausbeuten.

Die Morpholinderivate der Formel III sind bekannt und zum Teil im Handel erhältlich, oder können wie in Houben-Weyl, Methoden der Organischen Chemie, Band XI/1, S. 815 (1957) beschrieben, hergestellt werden.

Die erfindungsgemässen kristallinen Komplexverbindungen der Formel I eignen sich als Korrosionsinhibitoren in Ueberzugszusammensetzungen zum Schutz von metallischen Oberflächen. Als solche können sie allen flüssigen oder festen organischen Materialien zugesetzt werden.

Ein weiterer Gegenstand der Erfindung sind daher auch Ueberzugszusammensetzungen enthaltend a) ein organisches filmbildendes Bindemittel und b) als Korrosionsinhibitor mindestens eine kristalline Komplexverbindung der Formel I.

Bevorzugt ist die Ueberzugszusammensetzung ein Anstrichmittel. Speziell bevorzugt ist ein wässriges Anstrichmittel.

Anstrichmittel sind beispielsweise Lacke, Farben oder Firnisse. Diese enthalten stets ein organisches filmbildendes Bindemittel neben anderen fakultativen Komponenten.

Als organisches filmbildendes Bindemittel der Ueberzugszusammensetzung sind alle üblichen Filmbildner für lösungsmittelhaltige oder lösungsmittelfreie, insbesondere jedoch für wässrige Lackzusammensetzungen geeignet. Beispiele für solche Filmbildner sind Epoxidharze, Polyurethanharze, Aminoplastharze oder Mischungen solcher Harze; eine basische wässrige Dispersion oder eine Lösung eines sauren Harzes.

Bevorzugte organische filmbildende Bindemittel sind Epoxidharze, Polyurethanharze, Polyesterharze, Acrylharze und deren Copolymerharze, Polyvinylharze, Phenolharze, Alkydharze oder Mischungen von solchen Harzen.

Von besonderem Interesse sind organische filmbildende Bindemittel für wässrige Ueberzugszusammensetzungen wie z.B. Alkydharze; Acrylharze; 2-Komponenten-Epoxidharze; Polyurethanharze; Polyesterharze, welche üblicherweise gesättigt sind; wasserverdünnbare Phenolharze oder abgeleitete Dispersionen; wasserverdünnbare Harnstoffharze; Harze auf Basis von Vinyl-/Acrylcopolymeren.

Spezifischer betrachtet können die Alkydharze wasserverdünnbare Alkydharzsysteme sein, welche lufttrocknend oder in Form von Einbrennsystemen wahlweise in Kombination mit wasserverdünnbaren Melaminharzen eingesetzt werden können; es kann sich auch um oxidativ trocknende, lufttrocknende oder Einbrennsysteme handeln, welche wahlweise in Kombination mit wässrigen Dispersionen auf Basis von Acrylharzen oder deren Copolymeren, mit Vinylacetaten etc. angewandt werden.

Die Acrylharze können reine Acrylharze, Acrylsäureestercopolymere, Kombinationen mit Vinylharzen oder Copolymere mit Vinylmonomeren wie Vinylacetat, Styrol oder Butadien sein. Diese Systeme können lufttrocknende Systeme oder Einbrennsysteme sein.

Wasserverdünnbare Epoxidharze weisen in Kombination mit geeigneten Polyaminvernetzern ausgezeichnete mechanische und chemische Beständigkeit auf. Bei Verwendung von flüssigen Epoxidharzen kann auf einen Zusatz organischer Lösungsmittel zu wässrigen Systemen verzichtet werden. Die Anwendung von Festharzen oder Festharzdispersionen erfordert üblicherweise einen Zusatz geringfügiger Lösungsmittelmengen, um die Filmbildung zu verbessern.

Bevorzugte Epoxidharze sind solche auf Basis aromatischer Polyole, insbesondere auf Basis von Bisphenolen. Die Epoxidharze werden in Kombination mit Vernetzern angewandt. Bei letzteren kann es sich um insbesondere amino- oder hydroxyfunktionelle Verbindungen, eine Säure, ein Säureanhydrid oder eine Lewis-Säure handeln. Beispiele dafür sind Polyamine, Polyaminoamide, Polymere auf Basis von Polysulfiden, Polyphenole, Borfluoride und deren Komplexverbindungen, Polycarbonsäuren, 1,2-Dicarbonsäureanhydride oder Pyromellitsäuredianhydrid.

Polyurethanharze leiten sich von Polyethern, Polyestern und Polybutadienen mit endständigen Hydroxylgruppen einerseits und aliphatischen oder aromatischen Polyisocyanaten andererseits ab.

Geeignete Polyvinylharze sind beispielsweise Polyvinylbutyral, Polyvinylacetat oder deren Copolymere.

Geeignete Phenolharze sind Kunstharze, bei deren Aufbau Phenole die Hauptkomponente darstellen, also vorallem Phenol-, Kresol-, Xylenol- und Resorcin-Formaldehyd-Harze, Alkylphenolharze sowie Kondensationsprodukte aus Phenolen mit Acetaldehyd, Furfurol, Acrolein oder anderen Aldehyden. Von Interesse sind auch modifizierte Phenolharze.

Die Ueberzugszusammensetzungen können zusätzlich eine oder mehrere Komponenten aus der Gruppe der Pigmente, Farbstoffe, Füllstoffe, Fliesskontrollmittel, Dispergiermittel, Thixotropiemittel, Haftungsverbesserer, Antioxidantien, Lichtstabilisatoren oder Härtungskatalysatoren enthalten. Sie können auch noch andere bekannte Korrosionsschutzmittel enthalten, beispielsweise Korrosionsschutz-Pigmente, wie phosphat- oder borathaltige Pigmente oder Metalloxid-Pigmente, oder andere organische oder anorganische Korrosionsinhibitoren, z.B. Salze der Nitroisophthalsäure, Phosphorester, technische Amine oder substituierte Benztriazole.

Beispiele für zusätzliche Pigmente sind Titandioxid, Eisenoxid, Aluminiumbronze oder Phthalocyaninblau.

Beispiele für zusätzliche Füllstoffe sind Talk, Aluminiumoxid, Aluminiumsilikat, Baryt, Glimmer oder Siliciumdioxid.

Fliesskontrollmittel und Thixotropiemittel basieren etwa auf modifizierten Bentoniten.

Haftungsverbesserer basieren beispielsweise auf modifizierten Silanen.

Von Vorteil ist ferner der Zusatz von basischen Füllstoffen oder Pigmenten, die in bestimmten Bindemittelsystemen einen synergistischen Effekt auf die Korrosionsinhibierung bewirken. Beispiele für solche basischen Füllstoffe und Pigmente sind Calcium-oder Magnesiumcarbonat, Zinkoxid, Zinkcarbonat, Zinkphosphat, Magnesiumoxid, Aluminiumoxid, Aluminiumphosphat oder Gemische davon. Beispiele für basische organische Pigmente sind solche auf Basis von Aminoanthrachinon.

Die erfindungsgemässen Korrosionsinhibitoren können dem Anstrichmittel während dessen Herstellung zugesetzt werden, beispielsweise während der Pigmentverteilung durch Mahlen oder während des Extrudierprozesses im Falle von Pulverlacken, oder der Inhibitor wird in einem Lösungsmittel gelöst und anschliessend in die Ueberzugszusammensetzung eingerührt.

Die erfindungsgemässen kristallinen Komplexverbindungen der Formel I werden zweckmässig in einer Menge von 0,02 bis 20 Gew.-%, vorzugsweise 0,1 bis 10 Gew.-%, bezogen auf das Gewicht des Gesamtfestkörpers der Ueberzugszusammensetzung, verwendet.

Die Anstrichmittel können nach den üblichen Verfahren auf das Substrat aufgebracht werden, beispielsweise durch Sprühen, Tauchen, Streichen, elektrostatische Verarbeitung oder durch Elektroabscheidung. Oft werden mehrere Schichten aufgetragen. Die Korrosionsinhibitoren werden in erster Linie der Grundschicht (Primer) zugegeben, da sie vor allem an der Grenze Metall-Anstrich wirken. Sie können aber auch zusätzlich zur Zwischen-oder Deckschicht zugegeben werden. Je nachdem, ob das Bindemittel ein physikalisch, chemisch oder oxidativ trocknendes Harz oder ein hitze- oder strahlenhärtendes Harz ist, erfolgt die Härtung des Anstrichs bei Raumtemperatur oder durch Erwärmen (Einbrennen) oder durch Bestrahlung.

Vorzugsweise ist das Anstrichmittel ein Grundanstrich (Primer) für metallische Substrate, wie beispielsweise Eisen, Stahl, Kupfer, Zink oder Aluminium, sowie deren Legierungen.

Zusätzlich zur antikorrosiven Wirkung haben die erfindungsgemässen kristallinen Komplexverbindungen der Formel I den Vorteil, dass sie die Adhäsion Anstrich-Metall günstig beeinflussen und keine negativen Auswirkungen auf die Lagerstabilität der erfindungsgemässen Uebergangszusammensetzungen zeigen.

Eine bevorzugte Ausführungsform der vorliegenden Erfindung ist daher die Verwendung der kristallinen Komplexverbindungen der Formel I als Korrosionsinhibitoren in Ueberzugszusammensetzungen für metallische Oberflächen.

Die vorliegende Erfindung betrifft auch ein Verfahren zum Schutz eines korrodierbaren Metallsubstrats, das dadurch gekennzeichnet ist, dass man auf dieses eine Uebergangszusammensetzung aufbringt, die a) ein organisches filmbildendes Bindemittel und b) als Korrosionsinhibitor mindestens eine kristalline Komplexverbindung der Formel I enthält und sie anschliessend trocknet und/oder härtet.

Die folgenden Beispiele erläutern die Erfindung weiter. Angaben in Teilen oder Prozenten beziehen sich auf das Gewicht.

### Beispiel 1:

Herstellung des kristallinen Komplexes der 3-(4-Methylbenzoyl)propionsäure mit überschüssigem N-Ethylmorpholin [Verbindung (101)].

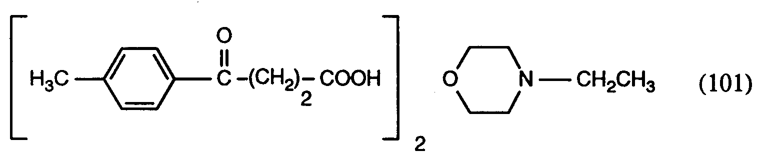

11,5 g (0,10 Mol) N-Ethylmorpholin und 9,60 g (0,05 Mol) 3-(4-Methylbenzoyl)propionsäure werden unter einer Stickstoffatmosphäre auf 60°C erwärmt. Die homogene, klare Reaktionslösung wird 10 Minuten bei 60°C gerührt und anschliessend unter Rühren auf Raumtemperatur abgekühlt. Der Niederschlag wird abfiltriert, mit Hexan mehrmals gut gewaschen und im Vakuumschrank bei 30°C getrocknet. Es resultieren 11,8 der Verbindung (101), weisse Kristalle, Smp. 66-68°C. Die Elementaranalyse entspricht der Zusammensetzung C₁₁H₁₂O₃ · 0,5 C₆H₁₃NO. Analyse berechnet: C 67,31; H 7,47; N 2,80 %. gefunden: C 67,57; H 7,39; N 2,55 %.

Von dieser Verbindung (101) wurde eine Röntgenstrukturanalyse durchgeführt. Die kristalline Komplexverbindung (101) schmilzt bei 66-68°C. Die Kristalle sind gross und oft verwachsen. Sie liegen als "milch-glas"-farbene Plättchen vor. Die dünneren Exemplare sind klarer. Für die Messung von einem grösseren Kristall wird mit Hilfe einer Rasierklinge ein optisch einwandfreies Teilstück abgetrennt und mit ®Araldit Rapid (Ciba Geigy) an eine MARK Glaskapillare geklebt. Das Kristall-Teilstück wird mit dem gleichen Klebstoff überzogen. Orientierungsaufnahmen (Polaroid) auf dem Precession Goniometer zeigen - für einen Kristall mit einem so niedrigen Schmelzpunkt - eine gute Reflexqualität und ein normales Streuvermögen. Die Elementarzelle, das Kristallsystem und die Raumgruppe werden auf dem Diffraktometer bestimmt. Während der 9 tägigen Messdauer bleibt der Kristall bis zum Schluss gut stabil.
Grösse des Einkristall-Teilstückes (mm): 0,90 x 0,72 x 0,20
Kristallsystem: monoklin Raumgruppe: P2₁/n (zentrosymmetrisch)
   - a = 28,466 Å: b = 9,623 Å c = 31,246 Å
   β = 111,73°
   Z = 4
   - Volumen = 7950,9 Å³: Dichte = 1,252 g/cm³ (berechnet)

Die Messungen wurden auf dem Philips Diffraktometer PW1100 mit MoKα₁-Strahlung (=0,70926 Å) durchgeführt.
- Reflexbreite:: 1,4°
- Messzeiten:: Hintergrund 2 x 10'', Reflexe 36 - 42''
- Messbedingungen:: Θ-2Θ-Scan-Verfahren, MoKα₁-Strahlung, 50 kV, 35 mA, Graphit-Monochromator
- Skalierung:: Durch Messen von 3 Referenzreflexen nach jeweils 2 Stunden.
- Messbereich:: Von 2Θ 6° - 44° wurden 10750 Reflexe vermessen. Davon wurden 3482 als beobachtet angenommen (I>2σ(I)).

### Beispiel 2:

Herstellung des kristallinen Komplexes der 3-(4-Methylbenzoyl)propionsäure mit unterschüssigem N-Ethylmorpholin [Verbindung (101)].

2,88 g (0,025 Mol) N-Ethylmorpholin und 9,60 g (0,05 Mol) 3-(4-Methylbenzoyl)propionsäure werden unter einer Stickstoffatmosphäre auf 70-75°C erwärmt Sobald eine klare Schmelze vorliegt, wird auf Raumtemperatur abgekühlt. Die feste Reaktionsmasse wird mit Hexan verrührt, filtriert, mit Hexan gewaschen und im Vakuumschrank bei 30°C getrocknet. Es resultieren 12,2 g der Verbindung (101), hellbraune Kristalle, Smp. 65-67°C. Die Elementaranalyse entspricht der Zusammensetzung C₁₁H₁₂O₃ · 0,5 C₆H₁₃NO. Analyse berechnet: C 67,31; H 7,47; N 2,80 %. gefunden: C 67,35; H 7,38; N 2,47 %.

### Beispiel 3:

Herstellung des kristallinen Komplexes der 3-(4-Methylbenzoyl)propionsäure mit überschüssigem N-Ethylmorpholin in Gegenwart von Hexan als Lösungsmittel [Verbindung (101)].

5,76 g (0,05 Mol) N-Ethylmorpholin und 4,8 g (0,025 Mol) 3-(4-Methylbenzoyl)propionsäure werden unter einer Stickstoffatmosphäre in 20 ml Hexan vorgelegt und auf Rückflusstemperatur erwärmt. Nach ca. 30 Minuten wird unter Rühren auf Raumtemperatur abgekühlt. Der Niederschlag wird abfiltriert, mit Hexan gewaschen und im Vakuumschrank bei 30°C getrocknet. Es resultieren 5,9 g der Verbindung (101), weisse Kristalle, Smp. 66-68°C. Die Elementaranalyse entspricht der Zusammensetzung C₁₁H₁₂O₃ · 0,5 C₆H₁₃NO. Analyse berechnet: C 67,31; H 7,47; N 2,80 %. gefunden: C 67,45; H 7,42; N 2,58 %.

### Beispiel 4:

Herstellung des kristallinen Komplexes der 3-(4-Methylbenzoyl)propionsäure mit N-Methylmorpholin [Verbindung (102)].

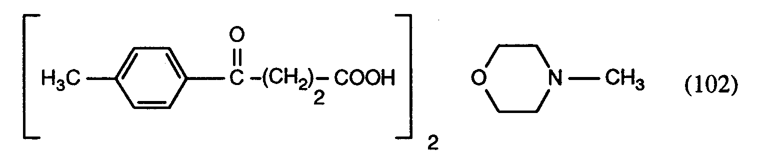

5,05 g (0,05 Mol) N-Methylmorpholin und 9,60 g (0,05 Mol) 3-(4-Methylbenzoyl)propionsäure werden unter einer Stickstoffatmosphäre auf 70°C erwärmt. Sobald eine klare Schmelze vorliegt, wird auf Raumtemperatur abgekühlt. Die feste Reaktionsmasse wird mit Hexan verrührt, filtriert, mit Hexan gewaschen und im Vakuumschrank bei 20°C getrocknet. Es resultieren 8,5 g der Verbindung (102), beige Kristalle, Smp. 67-68°C. Die Elementaranalyse entspricht der Zusammensetzung C₁₁H₁₂O₃ · 0,5 C₅H₁₁NO. Analyse berechnet: C 66,79; H 7,27; N 2,88 %. gefunden: C 66,54; H 7,30; N 2,88 %.

### Beispiel 5:

Herstellung des kristallinen Komplexes der 3-(4-Methylbenzoyl)propionsäure mit N-Isobutylmorpholin [Verbindung (103)].

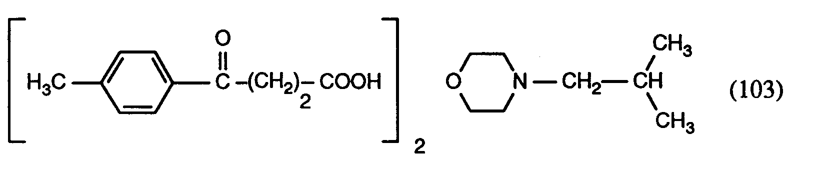

7,16 g (0,05 Mol) N-Isobutylmorpholin und 9,60 g (0,05 Mol) 3-(4-Methylbenzoyl)propionsäure werden unter einer Stickstoffatmosphäre auf 60°C erwärmt Die homogene, klare Reaktionslösung wird 10 Minuten bei 60°C gerührt und anschliessend unter Rühren auf Raumtemperatur abgekühlt. Der Niederschlag wird abfiltriert, mit Hexan mehrmals gut gewaschen und im Vakuumschrank bei 20°C getrocknet. Es resultieren 12,8 g der Verbindung (103), rosagelbe Kristalle, Smp. 69-71°C. Die Elementaranalyse entspricht der Zusammensetzung C₁₁H₁₂O₃ · 0,5 C₈H₁₇NO. Analyse berechnet: C 68,29; H 7,83; N 2,65 %. gefunden: C 68,28; H 7,81; N 2,55 %.

### Beispiel 6:

Herstellung des kristallinen Komplexes der 3-(4-Methylbenzoyl)propionsäure mit N-n-Butylmorpholin [Verbindung (104)].

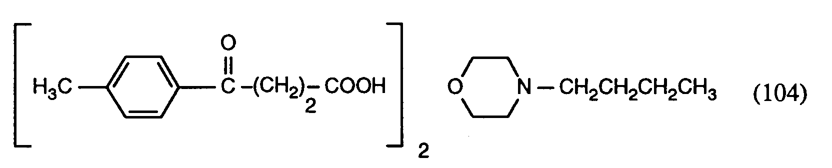

7,16 g (0,05 Mol) N-n-Butylmorpholin und 4,80 g (0,025 Mol) 3-(4-Methylbenzoyl)propionsäure werden unter einer Stickstoffatmosphäre auf 60°C erwärmt. Die homogene, klare Reaktionslösung wird 30 Minuten bei 60°C gerührt und anschliessend unter Rühren auf Raumtemperatur abgekühlt. Der Niederschlag wird abfiltriert, mit Hexan mehrmals gut gewaschen und im Vakuumschrank bei 20°C getrocknet. Es resultieren 5,6 g der Verbindung (104), beige Kristalle, Smp. 58-60°C. Die Elementaranalyse entspricht der Zusammensetzung C₁₁H₁₂O₃ · 0,5 C₈H₁₇NO. Analyse berechnet: C 68,29; H 7,83; N 2,65 %. gefunden: C 67,58; H 7,82; N 2,78 %.

### Beispiel 7:

Herstellung des kristallinen Komplexes der 3-(4-Chlorbenzoyl)propionsäure mit N-Ethylmorpholin [Verbindung (105)].

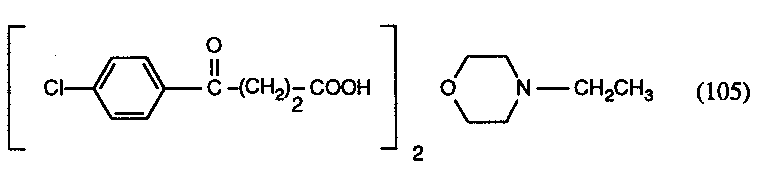

2,90 g (0,025 Mol) N-Ethylmorpholin und 5,30 g (0,025 Mol) 3-(4-Chlorbenzoyl)propionsäure werden unter einer Stickstoffatmosphäre auf 70°C erwärmt. Die homogene, klare Reaktionslösung wird 10 Minuten bei 60°C gerührt und anschliessend unter Rühren auf Raumtemperatur abgekühlt. Der Niederschlag wird abfiltriert, mit Hexan mehrmals gut gewaschen und im Vakuumschrank bei 20°C getrocknet. Es resultieren 6,0 g der Verbindung (105), weisse Kristalle, Smp. 77-79°C. Die Elementaranalyse entspricht der Zusammensetzung C₁₀H₉O₃Cl · 0,5 C₆H₁₃NO. Analyse berechnet: C 57,78; H 5,78; N 2,59; Cl 13,12 %. gefunden: C 57,88; H 5,75; N 2,51; Cl 13,22 %.

### Beispiel 8:

Herstellung des kristallinen Komplexes der 3-(4-Phenylbenzoyl)propionsäure mit N-Ethylmorpholin [Verbindung (106)].

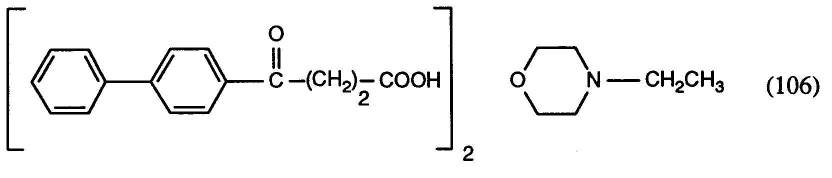

2,90 g (0,025 Mol) N-Ethylmorpholin und 3,20 g (0,025 Mol) 3-(4-Phenylbenzoyl)propionsäure werden unter einer Stickstoffatmosphäre auf 120°C erwärmt. Die homogene, klare Reaktionslösung wird 10 Minuten bei 120°C gerührt und anschliessend unter Rühren auf Raumtemperatur abgekühlt. Die feste Reasktionsmasse wird mit Hexan verrührt, filtriert, mit Hexan gewaschen und im Vakuumschrank bei 20°C getrocknet. Es resultieren 3,8 g der Verbindung (106), weisse Kristalle, Smp. 105-107°C. Die Elementaranalyse entspricht der Zusammensetzung C₁₆H₁₄O₃ · 0,5 C₆H₁₃NO. Analyse berechnet: C 73,17; H 6,63; N 2,25 %. gefunden: C 73,00; H 6,68; N 2,24 %.

### Beispiel 9:

Prüfung des kristallinen Komplexes der 3-(4-Methylbenzoyl)propionsäure mit N-Ethylmorpholin [Verbindung (101)] in wasserverdünnbarer 2K-Epoxidharz-Korrosionsschutzgrundierung auf Basis Beckopox EP 384 W / Beckopox EP 075 / Beckopox EH 623 W als Korrosionsinhibitor.

Zur Herstellung der Ueberzugszusammensetzung auf Basis Beckopox EP 384 W / Beckopox EP 075 / Beckopox EH 623 W werden die Komponenten 1 bis 8 (Formulierung ohne Additive) bzw. die Komponenten 1 bis 9 (Formulierung mit Additiv) in der angegebenen Reihenfolge eingesetzt (Komponente A, vgl. Tabelle 2).

**Tabelle 2**

| Wasserverdünnbare 2K-Epoxidharz-Korrosionsschutzgrundierung auf Basis Beckopox EP 384 W / Beckopox EP 075 / Beckopox EH 623 W | | |
|---|---|---|
| Zusammensetzung | Beispiel 9a Gew.-% | Beispiel 9b Gew.-% |
| **Komponente A:** | | |
| 1) Beckopox EH 623 W(80 % Lieferform)^{a)} | 14,4 | 14,4 |
| 2) Deion. Wasser | 29,2 | 29,2 |
| 3) Talkum AT Extra^{b)} | 13,8 | 13,8 |
| 4) Bayferrox 130 M^{c)} | 12,0 | 12,0 |
| 5) Millicarb^{d)} | 27,3 | 27,3 |
| 6) Bentone SD 2^{e)} | 0,70 | 0,70 |
| 7) Borchigel L 75 (25 % Lieferform)^{f)} | 1,70 | 1,70 |
| 8) Additol XL 270^{g)} | 0,9 | 0,9 |
| 9) Korrosionsinhibitor (Verbindung (101)) | ― | 3,10 |
| Total | 100,00 | 103,10 |

| **Komponente B:** | | |
|---|---|---|
| 10) Beckopox EP 384 W(54 % Lieferform)^{h)} | 63,0 | 63,0 |
| 11) Beckopox EP 075ⁱ⁾ | 3,7 | 3,7 |

| | | |
|---|---|---|
| a) ®Beckopox EH 623 W: Polyaminhärter (Hoechst AG); | | |
| b) ®Talkum AT Extra: Norwegian; | | |
| c) ®Bayferrox 130M: Eisenoxidrot (Bayer AG); | | |
| d) ®Millicarb: Calciumcarbonat (Omya); | | |
| e) ®Bentone SD 2: Antiabsetzmittel (Kronos Titan GmbH); | | |
| f) ®Borchigel L 75: Verdickungsmittel/Rheologieverbesserer (Gebr. Borchers AG); | | |
| g) ®Additol XL 270: Antiausschwimm-/Dispergierhilfsmittel (Hoechst AG); | | |
| h) ®Beckopox EP 384 W: Epoxidharz (Hoechst AG); | | |
| i) ®Beckopox EP 075: Reaktivverdünner (Polypropylenglykoldiglycidylether (Hoechst AG). | | |

Die resultierende Komponente A (Formulierung mit und ohne Korrosionsinhibitor) werden unter Verwendung einer Horizontalkugelmühle auf eine Kornfeinheit < 15 µm dispergiert. Das Disperdierergebnis wird durch Ermittlung des Grindometerwertes (ISO 1524) beurteilt.

Zur Applikation werden 100 g (Formulierung ohne Korrosionsschutzadditiv, Beispiel 9a) bzw. 103,1 g (Formulierung mit Korrosionsschutzadditiv, Beispiel 9b) mit 66,7 g der Komponente B gemischt. Zur Einstellung der gewünschten Spritzviskosität wird der Lack mit demineralisiertem Wasser verdünnt. Die Applikation erfolgt auf Stahlbleche (19 x 10,5 cm) des Typs Bonder (kalt gewalzter, entfetteter Stahl; Hersteller: Firma Chemetall, Frankfurt am Main / Deutschland) in einer Schichtdicke, welche nach dem Trocknen 60 µm beträgt (Trocknungsbedingungen: 10 Tage bei Raumpemperatur).

Vor Bewitterungsbeginn wird an den "Lackfilmen" unter Verwendung eines Bonder-Kreuzschnittgeräts (Mod. 205; Hersteller/Vertrieb: Firma Lau, Hemer/Deutschland) eine definierte Verletzung in Form eines Parallelschnitts (d.h. parallel zur längsten Blechkante) angebracht. Die Blechkanten werden durch Anbringen eines Kantenschutzes (®Icosit 255; Hersteller: Inertol AG, Winterthur, Schweiz) geschützt.

Die Proben werden im Anschluss einer Schnellbewitterung im Salzsprühtest (DIN 50 021 SS) während 190 Stunden bzw. einem Schwitzwassertest (ASTM D 4585-87) während 168 Stunden unterzogen. Die Resultate sind in den Tabelle 3 und 4 zusammengefasst. Die Bewertung der Resultate erfolgt basierend auf den relevanten DIN-Normen nach einem Bewertungsschlüssel durch Angabe eines Korrosionsschutzwertes CPF ("**C**orrosion **P**rotection **F**actor). Der CPF setzt sich additiv aus einer Beurteilung des Anstrichs (Film) und einer Beurteilung des Stahls zusammen und beträgt im Maximum 12 Punkte. Die Einzel-Maximalwerte für den Anstrich (Film) und den Stahl betragen 6 Punkte. Je grösser die Zahlen sind, desto besser ist der Korrosionsschutz.

**Tabelle 3**

| Salzsprühtest, 190 Stunden | | | |
|---|---|---|---|
| Ueberzugszusammensetzung | CPF Film | CPF Metall | CPF |
| Beispiel 9a | 2,4 | 1,3 | 3,7 |
| Beispiel 9b | 4,2 | 5,2 | 9,4 |

**Tabelle 4**

| Schwitzwassertest, 168 Stunden | | | |
|---|---|---|---|
| Ueberzugszusammensetzung | CPF Film | CPF Metall | CPF |
| Beispiel 9a | 2,8 | 2,0 | 4,8 |
| Beispiel 9b | 4,8 | 6,0 | 10,8 |

### Beispiel 10:

Prüfung des kristallinen Komplexes der 3-(4-Methylbenzoyl)propionsäure mit N-Ethylmorpholin [Verbindung (101)] in wässriger Dispersion auf Basis eines Acrylsäureester/Styrol-Copolymeren (Acronal S 760) als Korrosionsinhibitor.

Zur Herstellung der Ueberzugszusammensetzung auf Basis Acronal S 760 werden die Komponenten 1 bis 5 zunächst vorgemischt, danach erfolgt die Zugabe der Komponenten 6 und 7 (Formulierung ohne Korrosionsinhibitor) bzw. 6 bis 8 (Formulierung mit Korrosionsinhibitor, Verbindung (101), Beispiel 1) in der angegebenen Reihenfolge (vgl. Tabelle 5).

**Tabelle 5**

| Wässrige Dispersion auf Basis Acronal S 760 | | |
|---|---|---|
| Zusammensetzung | Beispiel 10a Gew.-% | Beispiel 10b Gew.-% |
| 1) Deion. Wasser | 8,20 | 8,20 |
| 2) Pigmentverteiler NL^{a)} | 0,15 | 0,15 |
| 3) Acronal S 760 (50 % Lieferform)^{b)} | 8,00 | 8,00 |
| 4) Shellsol D 60^{c)} | 1,00 | 1,00 |
| 5) Agitan 280^{d)} | 0,30 | 0,30 |
| 6) Millicarb^{e)} | 18,00 | 18,00 |
| 7) Bayferrox 130 M^{f)} | 10,00 | 10,00 |
| 8) Korrosionsinhibitor (Verbindung (101)) | ― | 1,14 |
| 9) Acronal S-760 (50 % Lieferform)^{b)} | 49,00 | 49,00 |
| 10) Agitan 280^{d)} | 0,30 | 0,30 |
| 11) Collacral PU 85/Butyldiglykol^{g)} | 4,10 | 4,10 |
| 12) Deion. Wasser | 0.95 | 0,95 |
| Total | 100,00 | 101,14 |

| | | |
|---|---|---|
| a) ®Pigmentverteiler NL: BASF AG; | | |
| b) ®Acronal S 760: Acrylsäureester-Styrol-Copolymer (wässrige Dispersion, BASF AG); | | |
| c) ®Shellsol D 60: Lackbenzin (Shell); | | |
| d) ®Agitan 280: Entschäumungsmittel (Münzing Chemie GmbH); | | |
| e) ®Millicarb: Calciumcarbonat (Firma Omya); | | |
| f) ®Bayferrox 130M: Eisenoxidrot (Bayer AG); | | |
| g) ®Collacral PU 85: Verdickungsmittel (BASF AG). | | |

Die resultierende Pigmentpaste wird mit einer Horizontalkugelmühle oder vergleichbarem auf eine Komfeinheit < 15 µm dispergiert. Die Kornfeinheit wird anhand des Grindometerwertes (ISO 1524) beurteilt.

Zur Fertigstellung des Lacks (Auflacken) werden anschliessend die Komponenten 9 bis 12 in der angegebenen Reihenfolge (Tabelle 5) zugegeben. Die Applikation erfolgt durch konventionelles Spritzen. Je nach gewünschter Viskosität kann der fertige Lack durch Zugabe von Butyldiglykol/deion. Wasser (1:1 g/g) verdünnt werden.

Die Applikation des Lacks erfolgt, wie in Beispiel 9 beschrieben, auf Stahlbleche des Typs Bonder in einer Schichtdicke, welche nach dem Trocknen 100 µm beträgt (Trocknungsbedingungen: 14 Tage bei Raumtemperatur).

Vor Bewitterungsbeginn wird an den "Lackfilmen" unter Verwendung eines Bonder-Kreuzschnittgeräts (Mod. 205; Hersteller/Vertrieb: Firma Lau, Hemer/Deutschland) eine definierte Verletzung in Form eines Parallelschnitts (d.h. parallel zur längsten Blechkante) angebracht. Die Blechkanten werden durch Anbringen eines Kantenschutzes (®Icosit 255; Hersteller: Inertol AG, Winterthur, Schweiz) geschützt.

Die Proben werden im Anschluss einer Schnellbewitterung im Salzsprühtest (DIN 50 021 SS) während 168 Stunden unterzogen. Die Resultate sind in Tabelle 6 zusammengefasst. Die Bewertung der Resultate erfolgt basierend auf den relevanten DIN-Normen nach einem Bewertungsschlüssel durch Angabe eines Korrosionsschutzwertes CPF ("**C**orrosion **P**rotection **F**actor). Der CPF setzt sich additiv aus einer Beurteilung des Anstrichs (Film) und einer Beurteilung des Stahls zusammen und beträgt im Maximum 12 Punkte. Die Einzel-Maximalwerte für den Anstrich (Film) und den Stahl betragen 6 Punkte. Je grösser die Zahlen sind, desto besser ist der Korrosionsschutz.

**Tabelle 6**

| Salzsprühtest (DIN 50 021 SS), 168 Stunden | | | |
|---|---|---|---|
| Ueberzugszusammensetzung | CPF Film | CPF Metall | CPF |
| Beispiel 10a | 2,4 | 2,6 | 5,0 |
| Beispiel 10b | 4,1 | 5,8 | 9,9 |

### Beispiel 11:

Prüfung des kristallinen Komplexes der 3-(4-Methylbenzoyl)propionsäure mit N-Ethylmorpholin [Verbindung (101)] in wässriger Acryldispersion (Maincote HG-54) als Korrosionsinhibitor.

Zur Herstellung der Ueberzugszusammensetzung auf Basis Maincote HG-54 werden die Komponenten 1 bis 8 (Formulierung ohne Korrosionsinhibitor) bzw. 1 bis 9 (Formulierung mit Korrosionsinhibitor) in der angegebenen Reihenfolge eingesetzt (vgl. Tabelle 7).

**Tabelle 7**

| Acryldispersion auf Basis Maincote HG-54 | |
|---|---|
| Zusammensetzung | Gew.-% |
| 1) Deion. Wasser | 3,10 |
| 2) Methylcarbitol ^{a)} | 5,00 |
| 3) Orotan 165 ^{b)} | 0,82 |
| 4) Triton CF 10 ^{c)} | 0,29 |
| 5) Drew Plus TS 4380 ^{d)} | 0,28 |
| 6) Acrysol RM 8 ^{e)} | 0,60 |
| 7) Bayferrox 130 M ^{f)} | 5,72 |
| 8) Millicarb ^{g)} | 17,40 |
| 9) erfindungsgemässer Korrosionsinhibitor | |
| 10) Butyldiglykol | 3,67 |
| 11) Maincote HG-54 ^{h)} | 58,70 |
| 12) Texanol ⁱ⁾ | 1,50 |
| 13) Dibutylphthalat ^{k)} | 1,50 |
| 14) Natriumnitrit (13,8 % in H₂O)^{l)} | 0,80 |
| 15) Drew T 4310 ^{m)} | 0,32 |
| 16) Ammoniaklösung (25 %) | 0,30 |
| Total | 100,00 |
| Gesamtfestkörper: 47 %; pH-Wert: 8 bis 8,5; | |

| | |
|---|---|
| a) ®Methylcarbitol: Diethylenglykolmonomethylether (Union Carbide); | |
| b) ®Orotan 165: Dispergierhilfsmittel (Rohm & Haas); | |
| c) ®Triton CF 10: nichtionisches Netzmittel (Rohm & Haas); | |
| d) ®Drew Plus TS 4380: Entschäumer (Drew Chem. Corp.); | |
| e) ®Acrysol RM 8: nichtionischer Verdicker (Rohm & Haas); | |
| f) ®Bayferrox 130 M: Eisenoxidrot (Bayer AG); | |
| g) ®Millicarb: Calciumcarbonat (Omya); | |
| h) ®Maincote HG-54: Acryldispersion, 41,5 % in deion. Wasser (Rohm & Haas); | |
| i) ®Texanol: Coalescent (Eastman Chem. Prod., Inc.); | |
| k) Dibutylphthalat: Weichmacher (Eastman Chem. Prod., Inc.); | |
| l) Natriumnitrit: Flugrostinhibitor (Fluka); | |
| m) ®Drew T 4310: nichtionischer Entschäumer (Drew Chem. Corp.). | |

Die Komponenten 1 bis 8 bzw. 1 bis 9 werden unter Verwendung eines Schnellrührers bei 3000 Umdrehungen/Minute auf eine Mahlfeinheit bzw. Mahlkörnigkeit von < 15 µm dispergiert. Das Dispergierergebnis der so erhaltenen Pigmentpaste wird durch Ermittlung des Grindometerwertes (ISO 1524) beurteilt. Die Einsatzmenge der erfindungsgemässen Korrosionsinhibitoren wird auf den Gesamtfestkörper der Formulierung ohne Additiv (Gesamtfestkörper: 47 %) bezogen. Demzufolge bedeutet beispielsweise Zusatz von 1 % Korrosionsinhibitor in 100 g Dispersion eine Menge von 0,47 g. Zur Fertigstellung der Ueberzugszusammensetzung werden die Komponenten 10 bis 16 gemäss Tabelle 7 bei reduzierter Rührgeschwindigkeit (1000 Umdrehungen/Minute) in der angegebenen Reihenfolge zugegeben. Anschliessend wird der pH-Wert der Formulierung kontrolliert und vor der Applikation gegebenenfalls mit Ammoniaklösung (25 %ig) auf einen Wert von pH 8 bis 8,5 nachgestellt.

Die Applikation der Ueberzugszusammensetzung kann unverdünnt durch Airless-Spritzen, Streichen, Rollen oder nach Verdunnung durch konventionelles Spritzen erfolgen. Die Verdünnung auf die gewünschte Spritzviskosität erfolgt durch Zugabe von Butylglykol/Wasser (1:1 g/g). Im vorliegenden Beispiel erfolgt die Applikation analog den Beispielen 9 bis 11 durch konventionelles Spritzen.

Die Applikation der Formulierung erfolgt auf Stahlbleche (19 mal 10,5 cm) des Typs Bonder (kalt gewalzter, entfetteter Stahl; Hersteller: Chemetall, Frankfurt am Main, Deutschland) in einer Schichtdicke, welche nach dem Trocknen 50-55 µm beträgt (Trocknungsbedingungen: 10 Tage bei Raumtemperatur).

Vor Bewitterungsbeginn wird an den "Lackfilmen" unter Verwendung eines Bonder-Kreuzschnittgeräts (Modell 205; Hersteller/Vertrieb: Firma Lau, 5870 Hemer/Deutschland) eine definierte Verletzung (70 mal 0,5 mm) in Form eines Parallelschnitts (d.h. parallel zur längsten Blechkante) angebracht. Die Blechkanten werden durch Anbringen eines Kantenschutzes (®Icosit 255; Hersteller: Inertol AG, Winterthur, Schweiz) geschützt.

Die Proben werden im Anschluss einer Schnellbewitterung im Salzsprühtest (DIN 50 021 SS) während 168 Stunden und im Schwitzwassertest (ASTM D 4585-87) während 330 Stunden unterzogen. Die Resultate sind in Tabelle 8 und 9 zusammengefasst. Die Bewertung der Resultate erfolgt basierend auf den relevanten DIN-Normen nach einem Bewertungsschlüssel durch Angabe eines Korrosionsschutzwertes CPF ("**C**orrosion **P**rotection **F**actor). Der CPF setzt sich additiv aus einer Beurteilung des Anstrichs (Film) und einer Beurteilung des Stahls zusammen und beträgt im Maximum 12 Punkte. Die EinzelMaximalwerte für den Anstrich (Film) und den Stahl betragen 6 Punkte. Je grösser die Zahlen sind, desto besser ist der Korrosionsschutz.

**Tabelle 8**

| Salzsprühtest, 168 Stunden | | | |
|---|---|---|---|
| Verbindung | CPF Film | CPF Metall | CPF |
| ― | 2,8 | 2,0 | 4,8 |
| 2 % (101) | 4,6 | 4,7 | 9,3 |
| 3 % (101) | 4,6 | 4,7 | 9,3 |

**Tabelle 9**

| Schwitzwassertest, 330 Stunden | | | |
|---|---|---|---|
| Verbindung | CPF Film | CPF Metall | CPF |
| ― | 3,0 | 0,6 | 3,6 |
| 2 % (101) | 2,2 | 5,8 | 8,0 |
| 3 % (101) | 3,0 | 5,8 | 8,8 |

### Beispiel 12:

Prüfung des kristallinen Komplexes der 3-(4-Methylbenzoyl)propionsäure mit N-Ethylmorpholin [Verbindung (111)] in wasserverdünnbarn Alkyd-/Urethan-Primern auf Basis Resydrol AZ 436 W / Daotan VTW 1237 als Korrosionsinhibitor.

Zur Herstellung der Ueberzugszusammensetzung auf Basis Resydrol AZ 436 W / Daotan VTW 1237 werden die Komponenten 1 bis 12 in der angegebenen Reihenfolge eingesetzt (vgl. Tabelle 10).

**Tabelle 10**

| Wasserverdünnbarer Alkyd- / Urethan-Primer auf Basis Resydrol AZ 436 W / Daotan VTW 1237 | |
|---|---|
| Zusammensetzung | Gew.-% |
| **Teil 1:** | |
| 1) Resydrol AZ 436 W (45 % Lieferform)^{a)} | 28,3 |
| 2) Ammoniaklösung (10 %) | |
| 3) Additol VXW 4940 (1:1 in Wasser) ^{b)} | 0,6 |
| Resydrol AZ 436 W wird mit der Ammoniaklösung auf pH = 8,5 neutralisiert, anschliessend wird Additol VXW 4930 eindispergiert. | |

| **Teil 2:** | |
|---|---|
| 4) Daotan VTW 1237 (32 % Lieferform)^{c)} | 39,8 |
| Teil 2 wird in Teil 1 eingerührt, anschliessend wird der pH-Wert kontrolliert und gegebenenfalls mit Ammoniaklösung auf pH 8,5 korrigiert. | |

| **Teil 3:** | |
|---|---|
| 5) Surfynol SE^{d)} | 0,3 |
| 6) Additol VXW 4973 ^{e)} | 0,3 |
| 7) Borchigel L 75 (25 % Lieferform) ^{f)} | 2,0 |
| 8) Mikrotalk AT Extra ^{g)} | 7,3 |
| 9) Bayferrox 130 M^{h)} | 6,4 |
| 10) Millicarbⁱ⁾ | 11,8 |
| 11) erfindungsgemässer Korrosionsinhibitor | |
| 12) Deion. Wasser | 3,2 |
| Total | 100,0 |

| | |
|---|---|
| a) ®Resydrol AZ 436 W: Alkydharzemulsion (Hoechst AG); | |
| b) ®Additol VXW 4940: Trockenstoff (Metalltrockner auf Basis Co, Zr, Ba)(Hoechst AG); | |
| c) ®Daotan VTW 1237: Polyurethanemulsion (Hoechst AG); | |
| d) ®Surfynol SE: Nichtionisches Netzmittel (Air Products and Chemicals); | |
| e) ®Additol VXW 4973: Antischaummittel (Hoechst AG); | |
| f) ®Borchigel L 75: Verdickungsmittel (Gebrüder Borchers AG); | |
| g) ®Microtalk AT Extra: mikronisierter Talk (Norwegian); | |
| h) ®Bayferrox 130 M: Eisenoxidrot (Bayer AG); | |
| i) ®Millicarb: Calciumcarbonat (Firma Omya). | |

Zur Herstellung der Ueberzugszusammensetzung auf Basis Resydrol AZ 436 W / Daotan VTW 1237 wird zunächst Resydrol AZ 436 W (Komponente 1) mit Ammoniaklösung (Komponente 2) auf pH = 8,5 gestellt. Anschliessend wird Komponente 3 gut eindispergiert. Daotan VTW 1237 (Komponente 4) wird im Anschluss in die Komponenten 1 bis 3 eingerührt, der pH-Wert wird kontrolliert und gegebenenfalls mit Ammoniaklösung auf pH = 8,5 nachgestellt. Danach erfolgt die Zugabe der Komponenten 5 bis 12 in der angegebenen Reihenfolge, wobei der Lackansatz lediglich andispergiert (sog. Andissolvern) wird. Nach stehen lassen über Nacht wird auf eine Kornfeinheit von < 15 µm dispergiert. Die erhaltene Kornfeinheit wird anhand des Grindometerwertes (ISO 1524) beurteilt. Die Einsatzmenge der erfindungsgemässen Korrosionsinhibitoren wird auf den Gesamtfestkörper der Formulierung ohne Korrosionsinhibitor (Gesamfestkörper: 51 %) bezogen. Demzufolge bedeutet beispielsweise ein Zusatz von 1 % Korrosionsinhibitor zu 100 g Primer eine Menge von 0,51 g.

Für konventionellles Spritzen wird der Lack mit deionisiertem Wasser auf die gewünschte Spritzviskosität eingestellt.

Die Applikation erfolgt auf Stahlbleche (19 x 10,5 cm) des Typs Bonder (kalt gewalzter, entfetteter Stahl; Hersteller: Firma Chemetall, Frankfurt am Main / Deutschland) in einer Schichtdicke, welche nach dem Trocknen 50 bis 55 µm beträgt (Trocknungsbedingungen: 14 Tage bei Raumtemperatur).

Vor Bewitterungsbeginn wird an den "Lackfilmen" unter Verwendung eines Bonder-Kreuzschnittgeräts (Mod. 205; Hersteller/Vertrieb: Firma Lau, Hemer/Deutschland) eine definierte Verletzung in Form eines Parallelschnitts (d.h. parallel zur längsten Blechkante) angebracht. Die Blechkanten werden durch Anbringen eines Kantenschutzes (®Icosit 255; Hersteller: Inertol AG, Winterthur, Schweiz) geschützt.

Die Proben werden im Anschluss einer Schnellbewitterung im Salzsprühtest (DIN 50 021 SS) während 120 Stunden unterzogen. Die Bestimmung der Korrosionsschutzwerte CPF erfolgt analog wie in Beispiel 11 beschrieben. Die Resultate sind in der Tabelle 11 zusammengefasst. Je grösser die Zahlen sind, desto besser ist der Korrosionsschutz.

**Tabelle 11**

| Salzsprühtest, 120 Stunden | | | |
|---|---|---|---|
| Verbindung | CPF Film | CPF Metall | CPF |
| ― | 2,0 | 2,5 | 4,5 |
| 2 % (101) | 3,8 | 4,5 | 8,3 |

## Patentansprüche

1. Kristalline Komplexverbindungen der Formel I 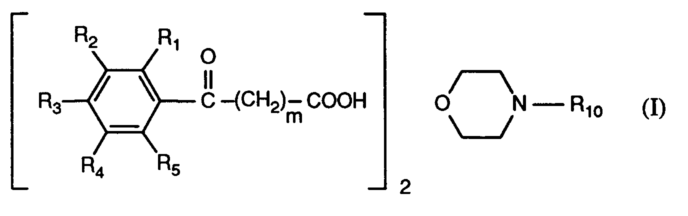 worin R₁, R₂, R₃, R₄ und R₅ unabhängig voneinander Wasserstoff, Halogen, Nitro, Cyano, CF₃, C₁-C₁₅-Alkyl, C₅-C₁₂-Cycloalkyl, C₂-C₁₅-Alkenyl, C₁-C₁₂-Halogenalkyl, C₁-C₁₂-Alkoxy, C₁-C₁₂-Alkylthio, unsubstituiertes oder durch C₁-C₄-Alkyl substituiertes C₆-C₁₀-Aryl; unsubstituiertes oder durch C₁-C₄-Alkyl substituiertes C₆-C₁₀-Aryloxy; unsubstituiertes oder am Arylrest durch 1 bis 3 C₁-C₄-Alkyl substituiertes C₇-C₁₂-Arylalkyl;
-CO₂R₆, -COR₆ oder 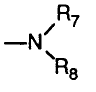 darstellen, wobei mindestens einer der Reste R₁ bis R₅ Wasserstoff, Halogen oder C₁-C₁₅-Alkyl bedeutet; ferner die Reste R₁ und R₂, R₂ und R₃, R₃ und R₄ oder R₄ und R₅ zusammen mit den Kohlenstoffatomen, an die sie gebunden sind, einen Benzo- oder Cyclohexenyl-Ring bilden,
R₆ C₁-C₂₀-Alkyl, durch Sauerstoff, Schwefel oder 〉N―R₉ unterbrochenes C₂-C₂₀-Alkyl;
unsubstituiertes oder am Arylrest durch 1 bis 3 C₁-C₄-Alkyl substituiertes C₇-C₁₂-Arylalkyl bedeutet,
R₇ und R₈ unabhängig voneinander Wasserstoff, C₁-C₂₄-Alkyl oder durch Sauerstoff,
Schwefel oder 〉N―R₉ unterbrochenes C₂-C₂₄-Alkyl darstellen,
R₉ Wasserstoff oder C₁-C₈-Alkyl bedeutet,
R₁₀ C₁-C₁₅-Alkyl, durch Sauerstoff, Schwefel oder 〉N―R₉ unterbrochenes C₂-C₂₀-Alkyl; C₅-C₁₂-Cycloalkyl, C₂-C₁₅-Alkenyl, C₁-C₁₂-Alkcoxy, C₁-C₁₂-Alkylthio, unsubstituiertes oder durch C₁-C₄-Alkyl substituiertes C₆-C₁₀-Aryl; unsubstituiertes oder durch C₁-C₄-Alkyl substituiertes C₆-C₁₀-Aryloxy; unsubstituiertes oder am Arylrest durch 1 bis 3 C₁-C₄-Alkyl substituiertes C₇-C₁₂-Arylalkyl darstellt, und
m eine ganze Zahl aus dem Bereich von 2 bis 5 bedeutet.

2. Kristalline Komplexverbindungen gemäss Anspruch 1, worin mindestens zwei der Reste R₁ bis R₅ Wasserstoff sind.

3. Kristalline Komplexverbindungen gemäss Anspruch 1, worin
R₁ Wasserstoff bedeutet,
R₂, R₃, R₄ und R₅ unabhängig voneinander Wasserstoff, Chlor, Brom, Nitro, Cyano, CF₃, C₁-C₈-Alkyl, C₅-C₇-Cycloalkyl, C₃-C₈-Alkenyl, C₁-C₈-Alkoxy, C₁-C₈-Alkylthio, Phenyl, Phenoxy, Benzyl, -CO₂R₆, -COR₆ oder 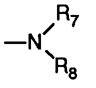 darstellen,
R₆ C₁-C₁₂-Alkyl, durch Sauerstoff unterbrochenes C₂-C₁₂-Alkyl; oder Benzyl bedeutet, und
R₇ und R₈ unabhängig voneinander Wasserstoff, C₁-C₈-Alkyl oder durch Sauerstoff unterbrochenes C₂-C₁₂-Alkyl darstellen.

4. Kristalline Komplexverbindungen gemäss Anspruch 1, worin
R₁₀ C₁-C₈-Alkyl, durch Sauerstoff oder Schwefel unterbrochenes C₃-C₁₂-Alkyl; C₅-C₇-Cycloalkyl, C₃-C₈-Alkenyl, C₁-C₈-Alkoxy, C₁-C₈-Alkylthio, Phenyl, Phenoxy oder Benzyl bedeutet.

5. Kristalline Komplexverbindungen gemäss Anspruch 1, worin
R₁, R₂ und R₄ Wasserstoff bedeuten,
R₃ und R₅ unabhängig voneinander Wasserstoff, Chlor, Brom, CF₃, C₁-C₈-Alkyl, Cyclohexyl, C₁-C₈-Alkoxy, Phenyl, -CO₂R₆, -COR₆ oder 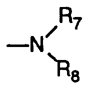 darstellen,
R₆ C₁-C₈-Alkyl bedeutet,
R₇ und R₈ unabhängig voneinander Wasserstoff oder C₁-C₈-Alkyl darstellen,
R₁₀ C₁-C₈-Alkyl, Cyclohexyl, Phenyl oder Benzyl darstellt, und
m eine ganze Zahl aus dem Bereich von 2 bis 4 bedeutet.

6. Kristalline Komplexverbindungen gemäss Anspruch 1, worin R₁, R₂, R₄ und R₅ Wasserstoff bedeuten, und m 2 ist.

7. Kristalline Komplexverbindungen gemäss Anspruch 1, worin R₁₀ C₁-C₄-Alkyl bedeutet.

8. Kristalline Komplexverbindungen gemäss Anspruch 1, worin
R₁, R₂, R₄ und R₅ Wasserstoff sind,
R₃ Wasserstoff, C₁-C₄-Alkyl, Phenyl oder Chlor bedeutet,
R₁₀ C₁-C₄-Alkyl darstellt, und
m 2 bedeutet.

9. Ueberzugszusammensetzung enthaltend
a) ein organisches filmbildendes Bindemittel und
b) als Korrosionsinhibitor mindestens eine kristalline Komplexverbindung der Formel I gemäss Anspruch 1.

10. Ueberzugszusammensetzung gemäss Anspruch 9, worin die Ueberzugszusammensetzung ein Anstrichmittel ist.

11. Ueberzugszusammensetzung gemäss Anspruch 9, worin die Ueberzugszusammensetzung ein wässriges Anstrichmittel ist.

12. Ueberzugszusammensetzung gemäss Anspruch 9, worin die Komponente a) ein Epoxidharz, ein Polyurethanharz, ein Polyesterharz, ein Acrylharz, ein Acrylcopolymerharz, ein Polyvinylharz, ein Phenolharz, ein Alkydharz oder eine Mischung solcher Harze ist.

13. Ueberzugszusammensetzung gemäss Anspruch 9, enthaltend zusätzlich eine oder mehrere Komponenten aus der Gruppe der Pigmente, Farbstoffe, Füllstoffe, Fliesskontrollmittel, Dispergiermittel, Thixotropiemittel, Haftungsverbesserer, Antioxidantien, Lichtstabilisatoren oder Härtungskatalysatoren.

14. Ueberzugszusammensetzung gemäss Anspruch 9, worin die Komponente b) in einer Menge von 0,02 bis 20 % bezogen auf das Gewicht des Gesamtfestkörpers der Ueberzugszusammensetzung vorliegt.

15. Verwendung der kristallinen Komplexverbindungen der in Anspruch 1 definierten Formel I als Korrosionsinhibitoren in Ueberzugszusammensetzungen für metallische Oberflächen.

16. Verfahren zum Schutz eines korrodierbaren Metallsubstrats, dadurch gekennzeichnet, dass man auf dieses eine Ueberzugszusammensetzung gemäss Anspruch 9 aufbringt und sie anschliessend trocknet und/oder härtet.

17. Verfahren zur Herstellung von kristallinen Komplexverbindungen der Formel I gemäss Anspruch 1, dadurch gekennzeichnet, dass eine Ketocarbonsäure der Formel II 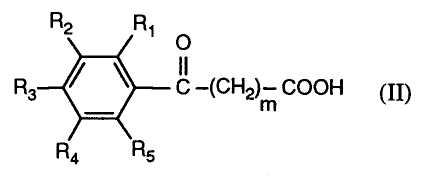 worin die allgemeinen Symbole wie in Anspruch 1 definiert sind, mit einem Morpholinderivat der Formel III 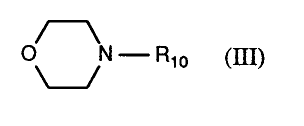 worin R₁₀ wie in Anspruch 1 definiert ist, ohne Lösungsmittel oder in Gegenwart eines aprotischen Lösungsmittels umgesetzt wird.

18. Verfahren gemäss Anspruch 17, dadurch gekennzeichnet, dass das molare Verhältnis der Ketocarbonsäure der Formel II zum Morpholinderivat der Formel III 4:1 bis 1:4 beträgt.

19. Produkt, erhältlich durch Umsetzung einer Ketocarbonsäure der Formel II 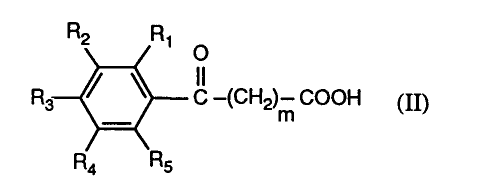 worin die allgemeinen Symbole wie in Anspruch 1 definiert sind, mit einem Morpholinderivat der Formel III 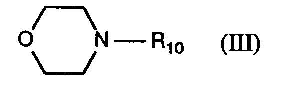 worin R₁₀ wie in Anspruch 1 definiert ist, ohne Lösungsmittel oder in Gegenwart eines aprotischen Lösungsmittels.

## Claims

1. A crystalline complex compound of the formula I 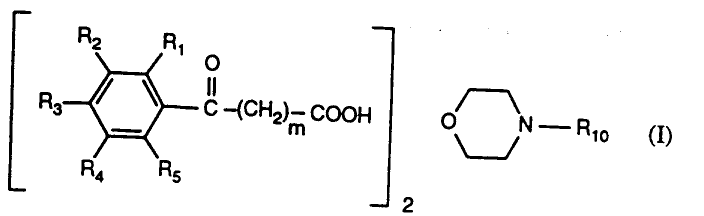 in which R₁, R₂, R₃, R₄ and R₅ are each independently of one another hydrogen, halogen, nitro, cyano, CF₃, C₁-C₁₅alkyl, C₅-C₁₂cycloalkyl, C₂-C₁₅alkenyl, C₁-C₁₂haloalkyl, C₁-C₁₂alkoxy, C₁-C₁₂alkylthio, unsubstituted or C₁-C₄alkyl-substituted C₆-C₁₀aryl; unsubstituted or C₁-C₄alkyl-substituted C₆-C₁₀aryloxy; unsubstituted C₇-C₁₂arylalkyl or C₇-C₁₂arylalkyl which is substituted in the aryl moiety by 1 to 3 C₁-C₄alkyl groups;
-CO₂R₆, -COR₆ or 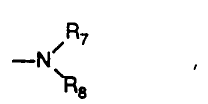 with the proviso that at least one of R₁ to R₅ is hydrogen, halogen or C₁-C₁₅alkyl; and the radicals R₁ and R₂, R₂ and R₃, R₃ and R₄ or R₄ and R₅, together with the carbon atoms to which they are attached, form a benzo or cyclohexenyl ring, R₆ is C₁-C₂₀alkyl, C₂-C₂₀alkyl which is interrupted by oxygen, sulfur or 〉N―R₉, unsubstituted C₇-C₁₂arylalkyl or C₇-C₁₂arylalkyl which is substituted in the aryl moiety by 1 to 32 C₁-C₄alkyl groups;
R₇ and R₈ are each independently of the other hydrogen, C₁-C₂₄alkyl or C₂-C₂₄alkyl which is interrupted by oxygen, sulfur or 〉N―R₉ ,
R₉ is hydrogen or C₁-C₈alkyl,
R₁₀ is C₁-C₁₅alkyl, C₂-C₂₀alkyl which is interrupted by oxygen, sulfur or 〉N―R₉;
C₅-C₁₂cycloalkyl, C₂-C₁₅alkenyl, C₁-C₁₂alkoxy, C₁-C₁₂alkylthio, unsubstituted or C₁-C₄alkyl-substituted C₆-C₁₀aryl; unsubstituted or C₁-C₄-alkyl substituted C₆-C₁₀aryloxy; unsubstituted C₇-C₁₂arylalkyl or C₇-C₁₂arylalkyl which is substituted in the aryl moiety by 1 to 3 C₁-C₄alkyl groups; and
m is an integer from 2 to 5.

2. A crystalline complex compound according to claim 1, wherein at least two of R₁ to R₅ are hydrogen.

3. A crystalline complex compound according to claim 1, wherein
R₁ is hydrogen,
R₂, R₃, R₄ and R₅ are each independently of one another hydrogen, chloro, bromo, nitro, cyano, CF₃, C₁-C₈alkyl, C₅-C₇cycloalkyl, C₃-C₈alkenyl, C₁-C₈alkoxy, C₁-C₈alkylthio, phenyl, phenoxy, benzyl, -CO₂R₆, -COR₆ or 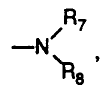 R₆ is C₁-C₁₂alkyl, C₂-C₁₂alkyl which is interrupted by oxygen; or benzyl, and
R₇ and R₈ are each independently of the other hydrogen, C₁-C₈alkyl or C₂-C₁₂alkyl which is interrupted by oxygen.

4. A crystalline complex compound according to claim 1, wherein
R₁₀ is C₁-C₈alkyl, C₃-C₁₂alkyl which is interrupted by oxygen or sulfur; C₅-C₇cycloalkyl, C₃-C₈alkenyl, C₁-C₈alkoxy, C₁-C₈alkylthio, phenyl, phenoxy or benzyl.

5. A crystalline complex compound according to claim 1, wherein
R₁, R₂ and R₄ are hydrogen,
R₃ and R₅ are each independently of the other hydrogen, chloro, bromo, CF₃, C₁-C₈alkyl, cyclohexyl, C₁-C₈alkoxy, phenyl, -CO₂R₆, -COR₆ or 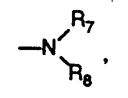 R₆ is C₁-C₈alkyl,
R₇ and R₈ are each independently of the other hydrogen or C₁-C₈alkyl,
R₁₀ is C₁-C₈alkyl, cyclohexyl, phenyl or benzyl, and
m is an integer from 2 to 4.

6. A crystalline complex compound according to claim 1, wherein R₁, R₂, R₄ and R₅ are hydrogen, and m is 2.

7. A crystalline complex compound according to claim 1, wherein R₁₀ is C₁-C₄alkyl.

8. A crystalline complex compound according to claim 1, wherein
R₁, R₂, R₄ and R₅ are hydrogen,
R₃ is hydrogen, C₁-C₄alkyl, phenyl or chloro,
R₁₀ is C₁-C₄alkyl, and
m is 2.

9. A coating composition comprising
a) an organic film-forming binder, and
b) as corrosion inhibitor, at least one crystalline complex compound of formula I according to claim 1.

10. A coating composition according to claim 9, wherein the coating composition is a paint or varnish.

11. A coating composition according to claim 9, wherein the coating composition is an aqueous paint or varnish.

12. A coating composition according to claim 9, wherein component a) is an epoxy resin, a polyurethane resin, a polyester resin, an acrylic resin, an acrylic copolymer resin, a polyvinyl resin, a phenolic resin, an alkyd resin, or a mixture of such resins.

13. A coating composition according to claim 9, which additionally comprises one or more components from the group consisting of pigments, dyes, fillers, flow control agents, dispersants, thixotropic agents, adhesion promoters, antioxidants, light stabilizers and curing catalysts.

14. A coating composition according to claim 9, which contains from 0.02 to 20% of component b), based on the weight of the total solids content of the coating composition.

15. The use of a crystalline complex compound of formula I as defined in claim 1 as corrosion inhibitor in a coating composition for metallic surfaces.

16. A method of protecting a corrodible metal substrate, which comprises applying to this substrate a coating composition according to claim 9 and subsequently drying and/or curing it.

17. A process for the preparation of a crystalline complex compound of formula I according to claim 1, which comprises reacting a ketocarboxylic acid of formula II 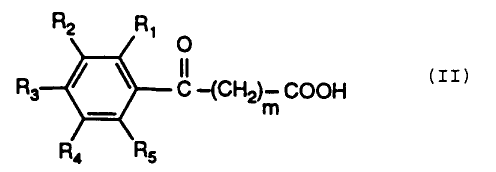 in which the general symbols are as defined in claim 1 with a morpholine derivative of formula III 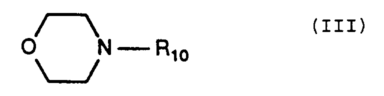 in which R₁₀ is as defined in claim 1, without solvent or in the presence of an aprotic solvent.

18. A processing according to claim 17, wherein the molar ratio of the ketocarboxylic acid of formula II to the morpholine derivative of formula III is from 4:1 to 1:4.

19. A product obtainable by reacting a ketocarboxylic acid of formula II 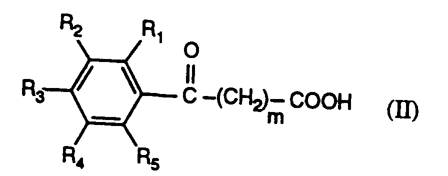 in which the general symbols are as defined in claim 1 with a morpholine derivative of formula III 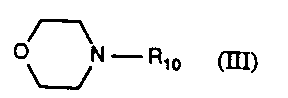 in which R₁₀ is as defined in claim 1, without solvent or in the presence of an aprotic solvent.

## Revendications

1. Composés complexes cristallins de formule I 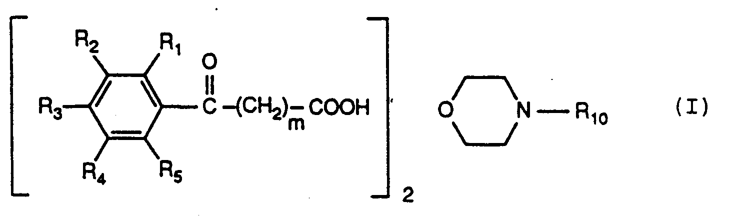 dans laquelle R₁, R₂, R₃, R₄ et R₅, indépendamment l'un de l'autre, représentent un atome d'hydrogène, un atome d'halogène, des groupes nitro, cyano, CF₃, alkyle en C₁-C₁₅, cycloalkyle en C₅-C₁₂, alcényle en C₂-C₁₅, halogénoalkyle en C₁-C₁₂, alcoxy en C₁-C₁₂, (alkyl en C₁-C₁₂)thio, aryle en C₆-C₁₀ non substitué ou substitué par alkyle en C₁-C₄ ; aryloxy en C₆-C₁₀ non substitué ou substitué par alkyle en C₁-C₄ ; arylalkyle en C₇-C₁₂ non substitué ou substitué sur le reste aryle par 1 à 3 groupes alkyle en C₁-C₄ ; -CO₂R₆, -COR₆ ou 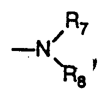 au moins l'un des restes R₁ à R₅ représente un atome d'hydrogène, un atome d'halogène ou un groupe alkyle en C₁-C₁₅ ; en plus, les restes R₁ et R₂, R₂ et R₃, R₃ et R₄, ou R₄ et R₅ ensemble avec les atomes de carbone auxquels ils sont liés, forment un cycle benzo- ou cyclohexényle,
R₆ représente un groupe alkyle en C₁-C₂₀, un groupe alkyle en C₂-C₂₀ interrompu par un atome d'oxygène, de soufre ou 〉N-R₉ ; un reste arylalkyle en C₇-C₁₂ non substitué ou substitué sur le reste aryle par 1 à 3 groupes alkyle en C₁-C₄ ;
R₇ et R₈ indépendamment l'un de l'autre représentent un atome d'hydrogène, un groupe alkyle en C₁-C₂₄, ou un groupe alkyle en C₂-C₂₄ interrompu par un atome d'oxygène, un atome de soufre ou 〉N-R₉ ;
R₉ représente un atome d'hydrogène ou un groupe alkyle en C₁-C₈,
R₁₀ représente un groupe alkyle en C₁-C₁₅, un groupe alkyle en C₂-C₂₀ interrompu par un atome d'oxygène ou un atome de soufre, ou 〉N-R₉ représente aussi des groupes cycloalkyle en C₅-C₁₂, alcényle en C₂-C₁₅, alcoxy en C₁-C₁₂, (alkyl en C₁-C₁₂)thio, aryle en C₆-C₁₀ non substitué ou substitué par alkyle en C₁-C₄; aryloxy en C₆-C₁₀ non substitué ou substitué par alkyle en C₁-C₄ ; arylalkyle en C₇-C₁₂ non substitué ou substitué sur le reste aryle par 1 à 3 groupes alkyle en C₁-C₄, et
m est un nombre entier de 2 à 5.

2. Composés complexes cristallins selon la revendication 1, où au moins deux des restes R₁ à R₅ représentent un atome d'hydrogène.

3. Composés complexes cristallins selon la revendication 1, où
R₁ représente un atome d'hydrogène,
R₂, R₃, R₄ et R₅, indépendamment l'un de l'autre, représentent des atomes d'hydrogène, de chlore, de brome, des groupes nitro, cyano, CF₃, alkyle en C₁-C₈, cycloalkyle en C₅-C₇, alcényle en C₃-C₈, alcoxy en C₁-C₈, (alkyl en C₁-C₈)-thio, phényle, phénoxy, benzyle, -CO₂R₆, -COR₆, ou 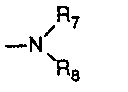
R₆ représente des groupes alkyle en C₁-C₁₂, alkyle en C₂-C₁₂ interrompu par un atome d'oxygène ; ou benzyle, et
R₇ et R₈, indépendaiment l'un de l'autre, représentent un atome d'hydrogène, des groupes alkyle en C₁-C₈ ou alkyle en C₂-C₁₂ interrompus par un atome d'oxygène.

4. Composés complexes cristallins selon la revendication 1, où
R₁₀ représente des groupes alkyle en C₁-C₈, alkyle en C₃-C₁₂ interrompus par des atomes d'oxygène ou de soufre ; des groupes cycloalkyle en C₅-C₇, alcényle en C₃-C₈, alcoxy en C₁-C₈, (alkyl en C₁-C₈)-thio, phényle, phénoxy ou benzyle.

5. Composés complexes cristallins selon la revendication 1, où
R₁, R₂, et R₄ représentent un atome d'hydrogène,
R₃ et R₅, indépendanment l'un de l'autre, représentent des atomes d'hydrogène, de chlore, de brome, des groupes CF₃, alkyle en C₁-C₈, cyclohexyle, alcoxy en C₁-C₈, phényle, -CO₂R₆, -COR₆, ou 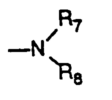
R₆ représente un groupe alkyle en C₁-C₈,
R₇ et R₈, indépendamment l'un de l'autre, représentent un atome d'hydrogène, ou un groupe alkyle en C₁-C₈,
R₁₀ représente des groupes alkyle en C₁-C₈, cyclohexyle, phényle ou benzyle ; et
m est un nombre entier de 2 à 4.

6. Composés complexes cristallins selon la revendication 1, où R₁, R₂, R₄ et R₅ représentent un atome d'hydrogène et m vaut 2.

7. Composés complexes cristallins selon la revendication 1, où R₁₀ représente un groupe alkyle en C₁-C₄.

8. Composés complexes cristallins selon la revendication 1, où
R₁, R₂, R₄ et R₅ représentent un atome d'hydrogène,
R₃ représene un atome d'hydrogène, des groupes alkyle en C₁-C₄, phényle ou un atome de chlore,
R₁₀ représente un groupe alkyle en C₁-C₄, et
m vaut 2.

9. Composition de revêtement contenant
a) un liant organique filmogène et
b) en tant qu'inhibiteur de corrosion, au moins un composé complexe cristallin de formule I, selon la revendication 1.

10. Composition de revêtement selon la revendication 9, où la composition de revêtement est une peinture.

11. Composition de revêtement selon la revendication 9, où la composition de revêtement est une peinture à l'eau.

12. Composition de revêtement selon la revendication 9, où le composant a) est une résine époxyde, une résine polyuréthanne, une résine polyester, une résine acrylique, une résine acrylique copolymère, une résine polyvinylique, une résine phénolique, une résine alkyde ou un mélange de ces résines.

13. Composition de revêtement selon la revendication 9, contenant de plus un ou plusieurs composants pris parmi les pigments, colorants, charges, régulateurs de fluidité, agents dispersants, agents de thixotropie, agents améliorant l'accrochage, antioxydants, photostabilisants ou catalyseurs de durcissement.

14. Composition de revêtement selon la revendication 9, dans laquelle le composant b) est présent dans une quantité de 0,02 à 20 % en poids par rapport au poids total de l'extrait sec de la composition de revêtement.

15. Utilisation des composés complexes cristallins de formule I définie dans la revendication 1, en tant qu'inhibiteurs de corrosion dans les compositions de revêtement de surfaces métalliques.

16. Procédé pour la protection d'un substrat métallique susceptible de corrosion, caractérisé en ce que l'on dépose sur celui-ci une composition de revêtement selon la revendication 9, que l'on sèche et/ou durcit ensuite.

17. Procédé pour la préparation de composés complexes cristallins de formule I selon la revendication 1, caractérisé ene ce que l'on fait réagir un acide cétocarboxylique de formule II 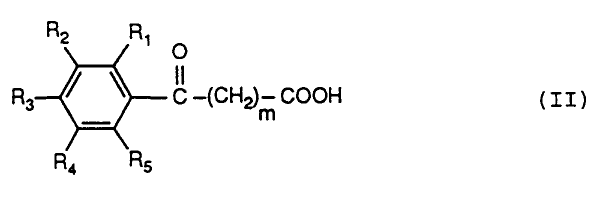 dans laquelle les symboles généraux sont définis comme dans la revendication 1, avec un dérivé de morpholine de formule III 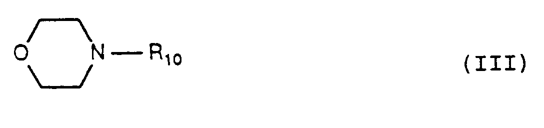 dans laquelle R₁₀ est défini comme dans la revendication 1, sans solvant ou en présence d'un solvant aprotique polaire.

18. Procédé selon la revendication 17, caractérisé en ce que la rapport molaire de l'acide cétocarboxylique de formule II au dérivé de morpholine de formule III est de 4:1 à 1:4.

19. Produit que l'on peut obtenir par réaction d'un acide cétocarboxylique de formule II 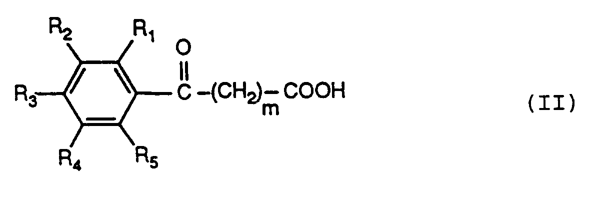 dans laquelle les symboles généraux sont définis comme dans la revendication 1, avec un dérivé de morpholine de formule III 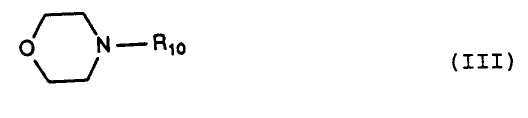 dans laquelle R₁₀ est défini comme dans la revendication 1, sans solvant ou en présence d'un solvant aprotique polaire.
